# EUROPEAN PATENT SPECIFICATION

(11) **EP 2 691 511 B1**
(45) Date of publication and mention of the grant of the patent: **19.08.2015**
(21) Application number: 12715878.0
(22) Date of filing: 29.03.2012
(51) Int. Cl.: C12N 5/00, G01N 33/50

(54) **METHOD FOR OBTAINING A MULTICELLULAR SPHEROID**
VERFAHREN ZUR HERSTELLUNG EINER MULTIZELLULÄREN SPHÄROIDS
PROCÉDÉ D'OBTENTION D'UN SPHÉROÏDE MULTICELLULAIRE

(30) Priority: 29.03.2011 GB 201105226
(43) Date of publication of application: 05.02.2014
(73) Proprietor: Universiteit Leiden, 2311 EZ Leiden (NL)
(72) Inventor: DANEN, Erik Hendrik Julius, NL-2333 CC Leiden (NL); DE SONNEVILLE, Jan, NL-2333 CH Leiden (NL); TRUONG, Hoa Hoang, NL-6525 GA Nijmegen (NL)
(74) Representative: Potter Clarkson LLP
(86) International application number: PCT/EP2012/055726
(87) International publication number: WO 2012/131000

(56) References cited:
- TROJANI C ET AL: "Three-dimensional culture and differentiation of human osteogenic cells in an injectable hydroxypropylmethylcellulose hydrogel", BIOMATERIALS, ELSEVIER SCIENCE PUBLISHERS BV., BARKING, GB, vol. 26, no. 27, 1 September 2005 (2005-09-01), pages 5509-5517, XP027768202, ISSN: 0142-9612 [retrieved on 2005-09-01]
- KELM J M ET AL: "Method for generation of homogeneous multicellular tumor spheroids applicable to a wide variety of cell types", BIOTECHNOLOGY AND BIOENGINEERING, WILEY & SONS, HOBOKEN, NJ, US, vol. 83, no. 2, 20 July 2003 (2003-07-20), pages 173-180, XP003018059, ISSN: 0006-3592, DOI: 10.1002/BIT.10655 cited in the application
- LINFEN YU ET AL: "Droplet-based microfluidic system for multicellular tumor spheroid formation and anticancer drug testing", LAB ON A CHIP, vol. 10, no. 18, 1 January 2010 (2010-01-01), page 2424, XP55032274, ISSN: 1473-0197, DOI: 10.1039/c004590j
- MICHAEL C W CHEN ET AL: "Alginate-based microfluidic system for tumor spheroid formation and anticancer agent screening", BIOMEDICAL MICRODEVICES, KLUWER ACADEMIC PUBLISHERS, BO, vol. 12, no. 4, 17 March 2010 (2010-03-17) , pages 647-654, XP019814139, ISSN: 1572-8781
- MARKOVITZ-BISHITZ Y ET AL: "A polymer microstructure array for the formation, culturing, and high throughput drug screening of breast cancer spheroids", BIOMATERIALS, ELSEVIER SCIENCE PUBLISHERS BV., BARKING, GB, vol. 31, no. 32, 1 November 2010 (2010-11-01), pages 8436-8444, XP027259549, ISSN: 0142-9612 [retrieved on 2010-08-07]
- NAPOLITANO ANTHONY P ET AL: "Scaffold-free three-dimensional cell culture utilizing micromolded nonadhesive hydrogels", BIOTECHNIQUES, INFORMA HEALTHCARE, US, vol. 43, no. 4, 1 October 2007 (2007-10-01), pages 494,496-500, XP002544941, ISSN: 0736-6205, DOI: 10.2144/000112591
- MADAYI P. NANDAKUMAR ET AL: "Superporous agarose monoliths as mini-reactors in flow injection systems", BIOSEPARATION, vol. 9, no. 4, 1 January 2000 (2000-01-01) , pages 193-202, XP55032330, ISSN: 0923-179X, DOI: 10.1023/A:1008117827057
- T W WONG ET AL: "Influence of polyvinylpyrrolidone on aggregation propensity of coated spheroids", INTERNATIONAL JOURNAL OF PHARMACEUTICS, vol. 242, 21 August 2002 (2002-08-21), pages 357-360, XP55032278, ISSN: 0378-5173, DOI: 10.1016%2FS0378-5173%2802%2900216-8
- HOA H TRUONG ET AL: "Automated microinjection of cell-polymer suspensions in 3D ECM scaffolds for high-throughput quantitative cancer invasion screens", BIOMATERIALS, ELSEVIER SCIENCE PUBLISHERS BV., BARKING, GB, vol. 33, no. 1, 21 September 2011 (2011-09-21), pages 181-188, XP028333969, ISSN: 0142-9612, DOI: 10.1016/J.BIOMATERIALS.2011.09.049 [retrieved on 2011-09-23] cited in the application

## Description

This invention relates to multicellular spheroids and their use in cellular assays. In particular, it relates to methods for generating multicellular spheroids for use in such assays.

The listing or discussion of a prior-published document in this specification should not necessarily be taken as an acknowledgement that the document is part of the state of the art or is common general knowledge.

Currently, chemical compound screens as well as RNAi screens for various types of cellular functions, including survival, growth, differentiation, and migration are principally performed in two-dimensional (2D) culture conditions. However, cells grown under such conditions have been shown to behave differently from the same cell types grown *in vivo.* Any of cell survival, proliferation, differentiation, cytoarchitecture, and migration may be altered on a 2D substrate (Bissel).

Accordingly, it is believed that the relevant microenvironment would be better mimicked by growing cells in three-dimensional (3D) cultures. Chemosensitivity in 3D cell culture has been shown to better mimic *in vivo* responses more carefully (Fischbach *et al,* 2007), and secretion of angiogenic factors by tumor cells in 3D cultures more closely resembles the *in vivo* situation as compared to 2D cultures (Claudia Fischbach, 2007).

Methods for culturing cells in 3D environments mainly involve forming compact aggregates which are subsequently placed in a 3D matrix. The best-known example of this approach is the hanging drop assay that was developed to create embryoid bodies from ES cells (Keller, 1995). In this method, multicellular spheroids (MSC) are generated by using the natural disposition of cells to aggregate at the bottom of a droplet. This method has also been used for non-embryonic cell types, including cancer cells to produce tumor-like structures (Kelm *et al,* 2002). Alternative methods involve mixing a single cell suspension with a solidifying extracellular matrix (ECM), resulting in individual cells eventually forming spheroids randomly within a 3D ECM structure (Lee *et al,* 2007), or by seeding polymeric scaffolds with cell/ECM suspensions (Fischbach *et al,* 2007).

Several of these 3D systems produce 3D cell aggregates wherein after compaction, depletion of oxygen, nutrients, and growth factors can occur in the core, leading to cell heterogeneity depending on the cell's position in the resulting multicellular spheroid (Mueller-Klieser, 1987; Sutherlands, 1988).

In 3D cultures, cell behaviour, including proliferation and migration is strongly affected by chemical (eg composition) and physical (eg rigidity, cross-linking) properties of the gel. Natural ECM proteins can be used such as collagen, fibrinogen, or the laminin-rich matrigel to represent the *in vivo* ECM composition most relevant to a given cell type. More recently, synthetic polymers have been developed that can be used to support 3D MCS culture environments (Loessner *et al,* 2010). Collagen type 1 is an abundant polymer in ECM *in vivo,* and it is widely used for 3D culture. It has been shown that various physical properties of the collagen gel, such as rigidity and pore size can have a major impact on stem cell differentiation, cancer growth, and cell migration (Buxboim and Discher, 2010; Friedl and Wolf, 2010; Leventhal *et al,* 2009). Cells can use various migration strategies in 3D environments, including mesenchymal or amoeboid individual cell migration modes or collective invasion strategies, depending on properties of the cells and of the matrix (Friedl and Wolf, 2010). Changes in matrix pore size can force cells to adopt alternative migration strategies or, if too extreme, pose a barrier to cell migration. Importantly, cells can modify the ECM by physical deformation and proteolysis, to overcome such barriers (Lammermann and Sixt, 2009).

Unfortunately, current methods to analyze cells in 3D are labour and time intensive and may create a high level of variability between experiments. Most of these methods such as the established hanging drop method follow a three-step protocol of cellular aggregation, leading to compaction of the spheroid, after which it can be transferred into a gel. This requires the use of specific cell types that are cohesive and are able to aggregate for example in the hanging drops. Moreover, differences in aggregation and compaction time even of suitable cell types, creates a strong need for optimisation for each cell type. As such, times to grow such spheroids and their ultimate size are highly variable and the procedure as a whole is difficult and time consuming.

Alternative methods in which single cell suspensions are created in ECM substrates that are subsequently allowed to form a gel are relatively easy to perform but also have several major disadvantages: formation of MCS depends on survival and proliferation of single cells in low adhesion conditions for extended periods; MCS formation is time consuming; MCS show a large variation in size; and MCS form at random locations, which is disadvantageous for imaging purposes.

Accordingly, there is a demand for MCS formation that is relatively fast and easy, highly reproducible, and that overcomes several of the disadvantages described above.

The inventors have now developed a novel method of culturing cells in 3D where cell suspensions are injected into a gel. By injecting the cells, the force exerted by the displaced gel is believed to move the cells into close proximity to each other. Further, the gel is thought to absorb the carrier fluid from the cell suspension so that the local cell concentration is increased still further. Both of these acts combine to encourage cells to form a coherent tissue structure.

The inventors have found that producing multicellular spheroids in this way has several advantages. Cell assays can begin immediately after injection and since there is no longer any requirement for cell culturing, the entire process may take a matter of hours rather than weeks. Injection of cell suspensions allows for the localization of the spheroids to be predetermined. Multiple cell types can be combined within a single multicellular spheroid and the method enables the formation of multiple multicellular spheroids, each having a distinct cellular composition, within a single gel. Due to the method's simplicity, it also lends itself to automation with very little human intervention. Moreover, some cell types grow better under the 3D conditions described herein. For instance, bone cells are difficult to culture in 2D but show better results in 3D culture.

Example 1 demonstrates the applicability of the method in high throughput screening efforts in a chemical screen for compounds that affect breast cancer invasion/migration. The method can easily be applied to cell suspensions derived directly from tumour biopsies. Example 2 describes the use of the method to study the regulation of tumour cell migration and metastatic potential by integrins.

A first aspect of the invention provides a method for producing a multicellular spheroid comprising injecting a cell suspension into a gel, wherein the cells are suspended in a carrier material.

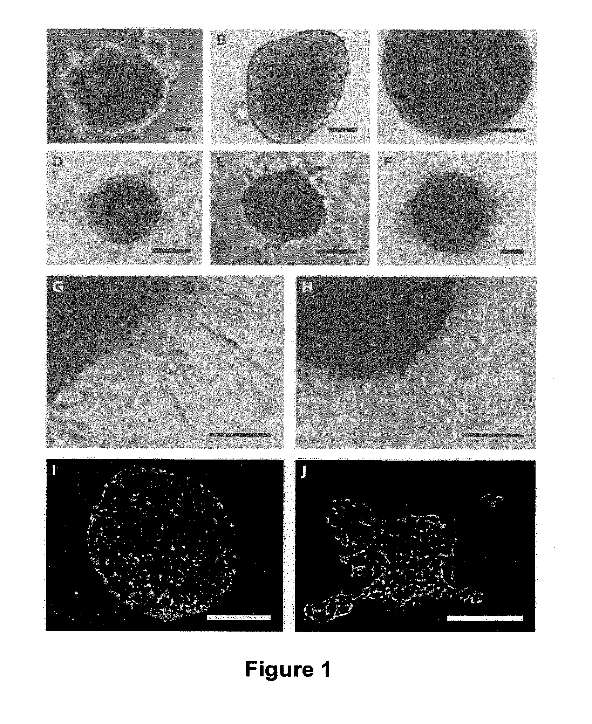

By a 'multicellular spheroid' we include the meaning of an aggregate, cluster or assembly of cells cultured to allow 3D growth in contrast to 2D growth of cells in either a monolayer or cell suspension (cultured under conditions wherein the potential for cells to aggregate is limited). The aggregate may be highly organised with a well defined morphology or it may be a mass of cells that have clustered or adhered together with little organisation reflecting the tissue of origin. For the avoidance of doubt, the spheroids shown in any of Figures 1, 2, 3, 5A-G, 6, 8, 9 (B5, C5, D5, E4, F4, G5, D7, D8, D9, D10, D11, G7, G8, G9, G10, G11), 11, 12, 13, 14, 16, 17, 18, 21E, 23D-3D, 23D-3D F-actin and 25 are considered to contain multicellular spheroids in accordance with the invention.

The multicellular spheroid may contain a single cell type (homotypic) or it may contain more than one cell type (heterotypic).

It is appreciated that the multicellular spheroid may be comprised of any one or more cell types, and that the determination of which cell types are used will depend on the application of the spheroid. For example, in optimising cancer drugs one may wish to produce a multicellular spheroid comprising cancer cells in order to assess the cytotoxicity of a candidate drug. In another experiment, optimising cancer drugs may involve determining the toxicity and selectivity of drug leads on multicellular spheroids comprising non-cancerous drugs. Correlation of the two experiments allows optimised lead compounds to be ranked according to their desirable toxicity to cancer cells versus undesirable toxicity to normal cells. Similarly, multicellular spheroids may comprise normal or transformed cells that can be used to screen for toxicity of drug candidates unrelated to cancer therapy or they may be used to assess particular properties of the cells as discussed below.

Suitable cells, or the tissue/organs they can be derived from, include bone marrow, skin, cartilage, tendon, bone, muscle (including cardiac muscle), blood vessels, corneal, neural, brain, gastrointestinal, renal, liver, pancreatic (including islet cells), lung, pituitary, thyroid, adrenal, lymphatic, salivary, ovarian, testicular, cervical, bladder, endometrial, prostate, vulval and esophageal.

Also included are the various cells of the immune system, such as T lymphocytes, B lymphocytes, polymorphonuclear leukocytes, macrophages and dendritic cells.

Also included are bacterial cells.

The cells may be stem cells, progenitor cells or somatic cells. Importantly, it is not a requisite for the cells to have the ability to form cell-cell contacts.

Preferably the cells are mammalian cells such as human cells or cells from animals such as mice, rats, rabbits, and the like.

In one embodiment, the invention does not make use of a human embryo for industrial or commercial purposes. The cells may be embryonic stem cells (eg human embryonic stem cells (totipotent or pluripotent)) which have been obtained by a method without involving the destruction of human embryos.

It is appreciated that the cells may be derived from a normal or healthy biological tissue, or from a biological tissue afflicted with a disease or illness, such as a tissue or fluid derived from a tumour.

In one embodiment, a cell suspension comprising cells from a biological sample taken from a subject is used to produce a multicellular spheroid. Hence, the cells may be derived from any of a biopsy, a surgical specimen, an aspiration, a drainage, or a cell-containing fluid. Suitable cell-containing fluids include any of blood, lymph, sebaceous fluid, urine, cerebrospinal fluid or peritoneal fluid. For example, in patients with transcoelomic metastasis, ovarian or colon cancer cells may be isolated from peritoneal fluid. Similarly, in patients with cervical cancer, cervical cancer cells may be taken from the cervix, for example by large excision of the transformation zone or by cone biopsy. Typically, such spheroids will contain multiple cell types that are resident in the tissue or fluid of origin. The cells may be obtained directly from the subject without intermediate steps of subculture, or they may first undergo an intermediate culturing step to produce a primary culture.

Methods for harvesting cells from biological tissue and/or cell containing fluids are well known in the art. For example, techniques used to obtain cells from biological tissue include those described by R. Mahesparan (Extracellular matrix-induced cell migration from glioblastoma biopsy specimens in vitro. Acta Neuropathol (1999) 97:231-239), M.O. Affia (The Growth and Cellular Kinetics of Human Cervical Cancer Spheroids in Relation to Drug Response. Eur J Cancer Clin Onc 22(9): 1095-1099, 1101-1103), Rolf Bjerkvig (Multicellular tumor spheroids from human gliomas maintained in organ culture J Neurosurg 72:463-475, 1990), and A. Corcoran (Evolution of the brain tumour spheroid model:transcending current model limitations. Acta Neurochir (2003) 145: 819-824), as well as those methods described in the Examples below.

In another embodiment, a cell suspension that comprises immortalised cells such as a cell line is used to produce a multicellular spheroid. Thus, the cells may be stable and highly passaged cell lines that have been derived from progenitor cells through many intermediate culture steps. The cell line may be a cancer cell line (e.g. a primary or metastatic cell line), or a non-cancer cell line. Examples of cancer cell lines include a prostate cancer cell line such as human LnCAP, Du145 and PC3; a breast cancer cell line such as any of human MDA MB-231, BT20, MDA-MB-435s, HCC1143, HCC1954, SUM149PT, SUM229PE, EVSA-T and SKBR7, and mouse 4T1; a melanoma cell line such as human MV3; a neuro-epithelial cell line such as mouse GE11; and a fibrosarcoma cell line such as human Ht1080. The panel of breast cancer cell lines described in Figure 1 of de Graauw et al, 2010 (PNAS 107(11): 6340-5).

Generally, the cells are first dissociated or separated from each other before forming the cell suspension. Dissociation of cells may be accomplished by any conventional means known in the art. Preferably, the cells are treated mechanically and/or chemically, such as by treatment with enzymes. By 'mechanically' we include the meaning of disrupting connections between associated cells, for example, using a scalpel or scissors or by using a machine such as an homogeniser. By 'enzymatically' we include the meaning of treating the cells with one or more enzymes disrupt connections between associated cells, including for example any of collagenase, dispases, DNAse and/or hyaluronidase. One or more enzymes may be used under different reaction conditions, such as incubation at 37°C in a water bath or at room temperature.

Preferably, the cells are treated to remove dead and/or dying cells and/or cell debris. The removal of such dead and/or dying cells is accomplished by any conventional means known to those skilled in the art, for example using beads and/or antibody methods. It is known, for example, that phosphatidylserine is redistributed from the inner to outer plasma membrane leaflet in apoptotic or dead cells. The use of Annexin V-Biotin binding followed by binding of the biotin to streptavidin magnetic beads enables separation of apoptotic cells from living cells. Similarly, removal of cell debris may be achieved by any suitable technique in the art, including, for example, filtration as described in the Examples below.

By 'cell suspension' we include the meaning of a sample of cells, including any of those mentioned above, suspended in a carrier material. Typically, the concentration of cells in the carrier material ranges from 1 million cells/30µl to 10 million cells/30µl. Preferably, the concentration of cells in the carrier material is between 7 and 10 million cells/30µl. For example, around 7 million cells/30µl is generally used for manual injection, while around 10 million cells/30µl is generally used for automated injection. Methods of determining cell concentration are known in the art, for example, the cells may be counted with a hemocytometer.

By 'carrier material' we include the meaning of a material that has a viscosity level that delays sedimentation of cells in a cell suspension, holds cells together after injection into a gel long enough for them to aggregate, and that is not too viscous so as to cause clogging in an injector.

As is widely known in the art, cells in suspension tend to sediment at a speed governed by the balance between viscous drag and gravity force. Thus, the carrier material must have sufficient viscosity to allow cells to remain suspended in the suspension at the point of injection. The viscosity required to achieve this can be optimised by the skilled person by monitoring the sedimentation rate at various viscosities and selecting a viscosity that gives an appropriate sedimentation rate for the expected time delay between loading the cell suspension in the injector and injecting into the gel. It is appreciated that some degree of sedimentation may be tolerated provided that the injector does not become clogged, as described below.

It is appreciated that the method involves the use of an injector, and that the carrier material has a viscosity that is not too viscous so as to cause clogging in the injector used. Whether a carrier material has the requisite viscosity that is not too viscous so as to cause clogging in an injector can be assessed by monitoring the injector tip when dispensing the cells. The viscosity of the carrier material should not be too high so that cells are retained on the tip of the injector instead of being dispensed, but rather the cells should flow out freely. It is understood that factors other than the viscosity of the carrier material may contribute to needle clogging and that the skilled person may need to optimise each factor so as to prevent clogging. For example, as described below, the aperture of the injector should be large enough to allow the cells to be dispensed without clogging. Also, large pieces of cell debris can cause injector clogging and so it is preferred if cell debris larger than the cells of the suspension, is removed prior to injection.

In one embodiment, the carrier material has a viscosity that allows at least some cells to remain suspended within a 5 cm length injector having a 60µm outer diameter (Eppendorf CustomTip Type III) for 30 minutes and which allows the cells to be subsequently dispensed freely without clogging of the injector.

Whether a carrier material has the requisite viscosity to allow cells to form a spheroid can be easily determined by injecting cells suspended in the carrier material into a gel and seeing whether a multicellular spheroid is formed. Assaying spheroid formation is routine in the art and may be done, for example, by microscopy and image analysis as described in Example 1. For example, cells may be stained for the cytoskeleton marker, actin and analysed with epi- or confocal microscopy, or with brightfield microscopy. Cell-cell contacts within the spheroid may be detected by staining cells with the cell-cell adhesion marker E-cadherin. Further methods are described herein. It is believed that the carrier material enables spheroid formation by its ability to trap cells during injection. Specifically, it is believed that the carrier material prevents cells from spreading directly in the gel during injection under the influence of the injection pressure. The concentration of cells in the injected aggregate is thought to be increased as the excess fluid within the injected droplet disperses into the surrounding gel.

In a preferred embodiment, the carrier material is one that comprises a polymer (e.g. a hydrophilic polymer), since the inventors believe that polymeric carrier materials have the requisite viscosity as discussed above. Preferably, when the carrier material comprises a polymer, the polymer chains are not crosslinked to each other. In any event, the carrier material is one that retains a fluid-like state.. It is particularly preferred if the polymer is non-immunogenic.

Examples of suitable polymers include polyvinylpyrrolidone (PVP), polyethylene glycol, dextran, Ficoll, Matrigel, polyhydroxyethyl methacrylate (PHEMA), polyvinyl alcohol (PVA), or polyethylene oxide (PEO) Further examples of appropriate polymers include any of those mentioned in Peppas et al, 2006, Adv Mater 18: 1345-1360).

Preferably, the carrier material comprises PVP. The inventors have tested carrier materials containing 1-8% (w/w ) PVP and so typically the carrier material contains 1-8% (w/w) polymer (eg PVP) such as about 1-6% (w/w) polymer (eg PVP), or about 1-4% (w/w) polymer (eg PVP), or about 1-3% (w/w) polymer (eg PVP), or about 1.5-2.5% (w/w) polymer (eg PVP). It is particularly preferred if the carrier material contains about 2% (w/w) polymer (eg PVP).

It is appreciated that the preferred concentration of polymer may vary depending on the type of polymer used. Also, depending upon the subject of study, one may wish to influence or not influence cell behaviour after injection using the carrier material. For example, when cancer cell migration is the subject of study, the concentration of carrier material relative to the concentration of the cells should be low enough not to prevent cell migration. Where the carrier material is one that is biodegradable by enzymes produced by the cells or other cellular processes, the concentration of the carrier material and cells will also effect how quick the carrier material degrades. Thus, the optimal concentration of carrier material may be affected by degradability, subject of study, cell type and cell concentration. In any event, the carrier material must be one that has the requisite viscosity level as defined above.

Generally, the carrier material is inert and does not chemically react with chemical compounds or compositions. In this way, the carrier material has no bioactivity and so does not affect survival, proliferation or differentiation of a cell.

However, it is appreciated that it may be desirable to add further components to the carrier material to render it bioactive. For example, any of an extracellular matrix protein (e.g. fibronectin), a drug (e.g. small molecules), a peptide, or an antibody may be added to the carrier material to modulate any of cell survival, proliferation or differentiation. The further component may be an inhibitor of a particular cellular function. Such 'bioactive' carrier materials may be used, for example, for drug delivery to the multicellular spheroid or to increase cell viability by reducing cell death and/or activation of cell growth/replication. Thus, the carrier material may further comprise components that are necessary for a cell's survival including any one or more of the following components: serum, interleukins, chemokines, growth factors, glucose, physiological salts, amino acids and hormones. For example, the carrier material may contain commercial culture media specific for a given cell type (eg as listed in the ATCC catalogue (www.atcc.org)). In one embodiment, the carrier material itself may be bioactive. For example, Matrigel comprises bioactive polymers that are important for cell viability, proliferation, development and migration.

In an embodiment, the carrier material contains a buffering agent to maintain the pH at a range of about 5-9, preferably about 6-8. Suitable buffers include sodium bicarbonate, phosphor-buffered saline and Hepes, or combinations thereof.

The gel can be any suitable gel known in the art, provided that it has sufficient mechanical stiffness to allow the formation of multicellular spheroids upon injection of a cell suspension into the gel. Thus, the gel must be able to withstand the injection pressure and hold the cell suspension in place to allow formation of a spheroid. Preferably, the gel is a hydrogel, by which we include the meaning of a polymer network that possesses a high water content so as to facilitate the transportation of oxygen, nutrients, waste and other soluble factors (Baroli, J Pharma Sci, 96(9): 2197). It is appreciated that unlike the carrier material which has a more liquid fluid state, the gel has a more solid rigid state.

Typically, the elastic modulus of the gel is in the range of about E=0.5 kPa to E=100 kPa, such as E=0.5-5 kPa, E=5-10 kPa, E=10-20 kPa, E=20-60 kPa or E=60-100 kPa. In a particular embodiment, the stiffness of the gel is in the range of 1-5 kPa so as to resemble the stiffness of normal tissue of around 1 kPa, or the stiffness of the gel is in the range of 5-10 kPa so as to resemble the stiffness of tumour tissue (eg around 5 kPa for soft tumours and 10 kPa for hard tumours). Methods of measuring elastic modulus of gels and how to adjust elastic modulus (e.g. by controlling extent of crosslinking) are standard practice in the art, and are described for example, in US 2010/0056390. Further methods of tailoring the molecular structure of gel are provided in Peppas et al (2006, Adv Mater 18: 1345-1360).

It is appreciated that the gel may or may not be crosslinked. Crosslinking of polymers to form a gel can be accomplished by any means known in the art, for example, by chemically mediated, ionically mediated, or thermally mediated crosslinking, or by photocrosslinking (Peppas et al (2006, Adv Mater 18: 1345-1360)). Crosslinking is important for mechanical stability, and controls the storage and release of soluble factors upon gel degradation. For crosslinked rubberlike gels, the elasticity is theoretically predicted to be related to the concentration of crosslinks by the relation G=nRT/V where n is the number of crosslinks per volume V and R is the gas constant (Yeung et al Cell Motil and the Cytoskeleton 60: 24-34).

In one embodiment, the gel is a natural gel. Thus, the gel may be comprised of one or more extracellular matrix components such as any of collagen, fibrinogen, laminin, fibronectin, vitronectin, hyaluronic acid, fibrin, alginate, agarose and chitosan.

In a preferred embodiment, the gel comprises collagen type 1 such as collagen type 1 obtained from rat tails. Collagen type 1 is easy to work, its solidification is less affected by temperature compared to other gel types, and it is believed to reflect the stromal environment of cells in tissues. The gel may be a pure collagen type 1 gel or may be one that contains collagen type 1 in addition to other components, such as other extracellular matrix proteins.

In another embodiment, the gel comprises Matrigel®, a reconstituted basement membrane mixture of laminin, collagen and other extracellular matrix proteins marketed by Becton Dickinson. The Matrigel® may be used in pure form or may be combined with other components (eg extracellular matrix proteins such as collagen).

Alternatively, the gel may be a synthetic gel. By a synthetic gel we include the meaning of a gel that does not naturally occur in nature. Examples of synthetic gels include gels derived from any of polyethylene glycol (PEG), polyhydroxyethyl methacrylate (PHEMA), polyvinyl alcohol (PVA), poly ethylene oxide (PEO).

Further examples of appropriate gels include any of those mentioned in Peppas et al (2006, Adv Mater 18: 1345-1360).

In any event, the composition of the gel should be one chosen so that at least one cell type after being injected into the gel is able to survive or grow. Any conventional method in the art may be used to assess cell survival or growth. Cell survival may be assessed by any of monitoring spheroid size, an Annexin V assay or a Caspase 3 assay, and cell growth may be assessed by measuring spheroid size. Where a mixture of cell types are injected into the gel (e.g. from primary cells), it is appreciated that the gel may cause cell death in specific cells while keeping others alive.

In one embodiment, for example when studying migration of cancer cells, it is preferred if the gel allows the cells to migrate effectively within the gel. When the cells are cancer cells, the aggressiveness of the cancer cells will affect their ability to migrate in a given cell type, and so it is preferred if the gel is one that allows migration of the cell type whose migration is to be studied. Migration of cells in multicellular spheroids may be assessed by any suitable method known in the art, including DIC image analysis as described in Examples 1 and 2. Other suitable techniques include phase contrast, bright-field, fluorescence and confocal imaging methods. However, it will be appreciated that other properties of the spheroid may be assessed, such as growth, without the need for cell migration.

It is appreciated that the desired composition of the gel can be determined by the skilled person and may vary depending on the type of cell to be injected into the gel and the eventual application of the spheroid. For example, MCF7 breast cancer cells survive well and can be stimulated to migrate in Matrigel® but do not grow or migrate well in pure collagen type 1 gels. In contrast, 4T1 breast cancer cells and PC3 prostate cancer cells grow well in pure collagen type 1 gels but not in Matrigel®. Other cells can survive, grow and invade/migrate efficiently in various gel types, such as HT1080 fibrosarcoma cells in fibrinogen, collagen or Matrigel® based gels. MDA-MB-231, HMT-3522, S-1 and T4-2 breast epithelial and breast cancer cells are known to survive in Matrigel or EHS-laminin derived gels (J Cell Biol 137:231, 1997; Cancer Res 66: 1526, 2006).

The gel may comprise one or more component at various concentrations provided that the gel has the requisite properties described above. Thus, for collagen type 1 gels, the gel typically comprises 0.7-2.5 mg/ml collagen type 1, such as between 0.7-1/0 mg/ml or between 1.0-1.5 mg/ml or between 1.5-2.0 mg/ml or between 2.0-2.5 mg/ml collagen type 1. For instance, the gel may comprise 0.7, 0.8, 0.9, 1.0, 1.1, 1.2, 1.3, 1.4, 1.5, 1.6, 1.7, 1.8, 1.9, 2.0, 2.1, 2.2, 2.3, 2.4 or 2.5 mg/ml collagen type 1. It is appreciated that the precise concentration used may require adjustment to account for variations between different collagen stock solutions or different commercial batches of collagen.

It is appreciated that the concentration of the one or more components in the gel may affect the ability of cells to survive, grow and migrate in the gel, and so the concentration may vary according to cell to be injected. For example, for collagen type 1 gels, 1.5 mg/ml collagen type 1 is the optimum concentration for 4T1 breast cancer cells to show invasive/migratory behaviour whereas 1 mg/ml collagen type 1 is the optimum concentration for PC3 prostate cancer cells to grow and invade/migrate. The skilled person can readily optimise the concentration of the one or more components in the gel for a particular cell type, for example by injecting a cell suspension into various gels with different component (eg collagen/PBS) concentrations as described in Example 1.

Conveniently, the gel comprises a growth medium that is able to provide components necessary for the survival of cells. The growth medium may be included as a diluent during gel formation or may be added after gel formation. The growth medium may comprise one or more of the following components: serum, buffer, interleukins, chemokines, growth factors, hydrogen carbonate, glucose, physiological salts, amino acids and hormones. Preferred mediums include RPMI 1640 medium (invitrogen) and DMEM medium (Invitrogen), preferably supplemented with blood serum such as fetal bovine serum. The medium may further comprise additional components such as antibiotics. It is appreciated that the desired growth medium can be determined by the skilled person and may vary depending on the cells to be injected. For example, human prostate cancer cell lines such as LnCAP, Du145 and PC3 and mouse breast cancer cell lines such as 4T1 grow best in complete RPMI 1640 medium, whereas human melanoma cell lines such as MV3, human breast cancer cell lines such as MDA MB-231, mouse neuro-epithelial cell lines such as GE11 and human fibrosarcoma cell lines such as Ht1080, grow best in DMEM medium. Growth media specifically suited to individual cell lines can be identified using the ATCC catalogue (www.atcc.org).

In a further embodiment, the gel contains a buffering agent to maintain the pH at a range of about 5-9, preferably about 6-8. Suitable buffers include sodium bicarbonate, phosphor-buffered saline and Hepes, or combinations thereof.

In another embodiment, the gel may contain one or more bioactive compounds, such as any of an extracellular matrix protein (e.g. fibronectin), a drug (e.g. small molecules), a peptide, a growth factor, or an antibody. For example, the gel may be modified with one or more cell adhesion peptides eg. RGD, YIGSR or derivatives thereof which are well known in the art and are commercially available. RGD is a tripeptide that is a versatile cell recognition site for numerous adhesive proteins (eg fibronectin) and is involved in cell binding. Methods for modifying gels by linking them to bioactive compounds such as cell adhesion molecules (e.g. by physical or chemical entrapment) are well known in the art, for example in US patent no 7,186,413 and in Peppas et al (2006, Adv Mater 18: 1345-1360).

It is appreciated that any carrier material described herein may be used in combination with any gel described herein, in the method of the invention. Preferably, the carrier material is less viscous and more fluid like that the gel. When the gel is crosslinked, it is preferred if the carrier material has less crosslinking than the gel and most preferably no crosslinking. Where the carrier material and gel comprise the same polymer, generally the concentration of the polymer in the gel is higher than the concentration of the polymer in the carrier material.

In a particularly preferred embodiment, the carrier material comprises PVP and the gel is a collagen gel.

The injection may take place by any means known in the art. Typically, the injection means is in the form of a micropipette having a sharp tip (e.g. a glass capillary or needle). The aperture of the injector should be large enough to allow cells to be dispensed freely but not too large so as to cause clogging of the injector with the gel. Generally, the aperture is between 10-100 micrometer such as between 20-100 micrometer, 40-80 micrometer and 50-70 micrometer. A preferred injector is an Eppendorf CustomTip Type III, with an outer diameter of 60 micrometer, Front surface 40 and flexibility: rigid. When bacterial cells are injected, it is appreciated that the inner injector diameter is smaller, typically between 10 and 15 micrometer, preferably around 10 micrometer.

Typically, volumes of cell suspensions in the nl to microliter range are injected into the gel, depending on the size of spheroid that is desired. For example, as described in Example 1, the inventors injected 80 nl cell suspension into a gel resulting in spheroid formation within one hour. Thus, in a preferred embodiment 10-200 nl cell suspension is injected into the gel, such as no more than 190 nl, 180 nl, 170 nl, 160 nl, 150 nl, 140 nl, 130 nl, 120 nl, 110 nl, 100 nl, 90 nl, 80 nl, 70 nl, 60 nl, 50 nl, 40 nl, 30 nl, or 20 nl cell suspension. In a particularly preferred embodiment, about 20-100 nl cell suspension is injected into the gel, such as about 40 nl, 50 nl, 60 nl, 70 nl, or 80 nl cell suspension.

By adjusting the volume of suspension injected and/or the concentration of cells in the suspension, the size of the multicellular spheroid can be tailored. For example, the inventors have shown that injecting 40 nl of cell suspension (~7 million cells/30 microliter carrier material) produces a multicellular spheroid of around 300 micrometer in diameter, and so to obtain spheroids of larger diameter the cell concentration and/or volume of cell suspension may be increased. Spheroids of between 200-500 micrometer in diameter are particularly suited to assessing tumour characteristics such as necrotic centres.

In one embodiment, the method comprises producing multiple multicellular spheroids by injecting at different sites in one or more gels. Thus, the method may comprise producing at least 2, 3, 4, 5, 6, 7, 8, 9, 10, 20, 30, 40, 50, 60, 70, 80, 90 or 100 multicellular spheroids or more. It is appreciated that different cell suspensions may be injected at different sites in one or more gels so as to produce multicellular spheroids having different cell compositions. This may be useful, for example, in assessing the effect of a particular drug candidate on a variety of different cell types. It is also appreciated that the multiple multicellular spheroids may be produced in a single gel or in respective gels, for example in different wells of a multi-well plate. In certain circumstances, it may be desirable for the respective gels to have different compositions, for example to assess the effect of different bioactive agents present in the respective gels on cell biology.

In one embodiment, the cell suspension is manually injected. For example, the cell suspension may be injected into the gel using a microinjector such as a 20 psi, PV280 pneumatic PicoPump as described in Example 1. However, it is appreciated that the method has the potential to produce multicellular spheroids with high reproducibility on a large scale, and so in an alternative embodiment, the cell suspension is automatically injected. Injection may be accomplished, for example, by simple repetitive and coordinated computer control of a stage positioner, micromanipulator and pressure unit.

Accordingly, it will be understood that the method of the first aspect of the invention may be automated or semi-automated to allow high-throughput production of multicellular spheroids. For example, the method may be carried out using an apparatus that comprises one or more injectors that can inject respective cell suspensions into gel located on a platform. It will be appreciated that either the one or more injectors may be stationary and the platform is movable with respect to the injectors, or that the one or more injectors are movable with respect to the platform. Such an apparatus may be robotic and/or computer controlled.

Conveniently, the multicellular spheroids are produced in gel on a multi-well plate (such as 96, 384, 1536 well plates and the like). In this way, a large number of multicellular spheroids can be produced and a large number of 3D cell assays can be performed simultaneously. Preferably, the multi-well plate is transparent which allows for imaging of the multicellular spheroid to be obtained from underneath the spheroid through the bottom of the plate.

In one embodiment, a single multicellular spheroid is formed in each well of the multi-well plate by injecting a single cell suspension in each well. This allows the effect of various agents on a multicellular spheroid to be assayed, for example by subjecting each well to a different agent. Alternatively, it may be desirable to form more than one multicellular spheroid in a gel in each well, such as 2 or 3 or 4 or 5 or 6 or 7 multicellular spheroids. The more than one multicellular spheroids formed within the well may have different cell compositions, for example derived from different tissue types, which may be useful in assaying how different cells interact with each other, eg how they affect each others growth, survival and/or migration. In a particular example, pro-angiogenic properties of cancer cells may be studied by combining a 'cancer cell' multicellular spheroid with an 'endothelial cell' multicellular spheroid, and the system may be used to screen for anti-angiogenic drugs.

It is appreciated that by injecting cell suspensions into a gel, multicellular spheroids may be formed at predetermined locations. For example, particularly when the method is automated, multicellular spheroids can be formed at predetermined distances from each other. The spheroids may be formed at regular or non-random spaced intervals or a pattern of multicellular spheroids may be produced (e.g. in rows and columns, or in a hexagonal pattern). Multicellular spheroids may be formed at the same position in each well of a multi-well plate.

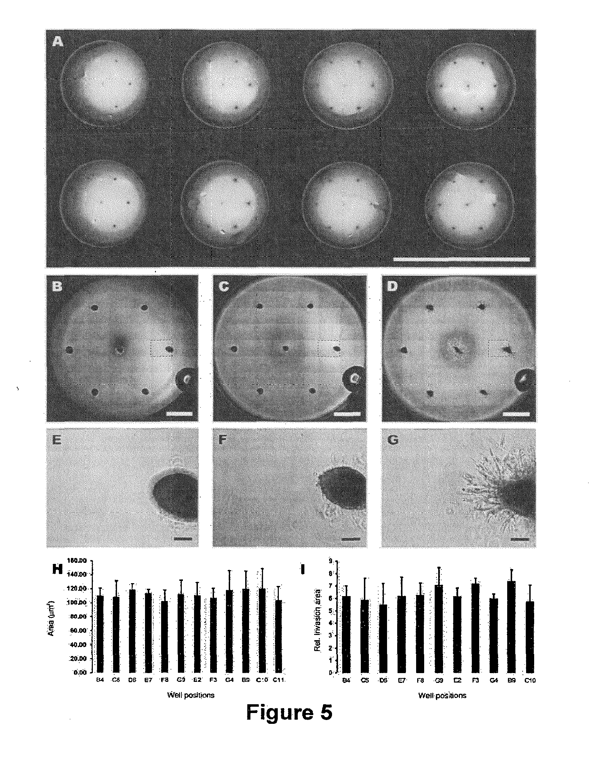

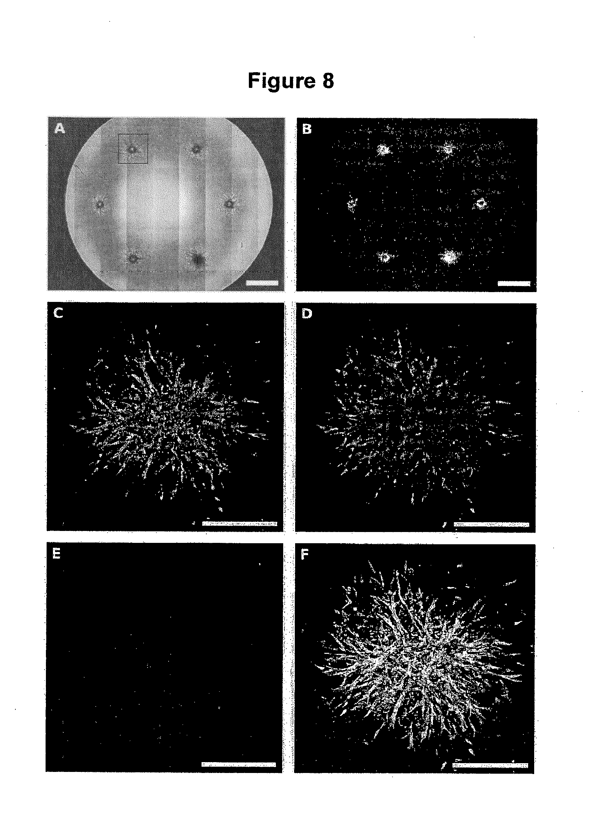

As described in Example 1, the inventors noted that a hexagonal pattern of 19 spheroids spaced at 1.2 mm apart showed interaction between spheroids, and so, for screening purposes, chose a less dense hexagon pattern of 6 or 7 spheroids spaced at 2 mm (e.g. Figures 5 and 8). This allowed sufficient space for outgrowth during the first 4 days. Accordingly, when multiple multicellular spheroids are produced in a single gel, the spheroids may be formed at least 2 mm apart, such as at least 2.5, 3.0, 3.5, 4.0, 4.5 or 5.0 mm apart. However, it may be desirable to study interactions between spheroids in which case the spheroids may be formed less than 2 mm apart, such as about 1.2 mm apart. It is appreciated that the amount and density of the gel surrounding the injection point both influence formation of a multicellular spheroid following injection. Thus, to increase reproducibility, the injection points are preferably chosen to be similar in gel density (e.g. by using the same injection height) and similar in gel volume (e.g. by keeping the distance to the edge of the gel the same). For this reason, in multiwell plates, the distance between the point of injection and the edge of the well is preferably kept constant. For example, spheroids located in the middle of the gel around the gel meniscus tend to be less reproducible than spheroids equidistantly located from the well edges.

Since cell suspensions can be injected into a gel at one or more predetermined locations, the locations of the resultant multicellular spheroids are known. Analysis of the multicellular spheroids is thereby facilitated since cell cultures no longer need to be located in a gel, for example by using optical guidance systems, but the analysis can be directly targeted at known locations. This is especially important when the analysis of the resultant spheroids is automated, since images of predetermined locations can be immediately taken once the cell suspensions have been injected and the multicellular spheroids formed.

A second aspect of the invention provides a method of producing a gel comprising one or more multicellular spheroids, the method comprising injecting a cell suspension into a gel, wherein the cells are suspended in a carrier material.

Preferences for the gel, cell suspension, carrier material and resultant multicellular spheroids include those defined above with respect to the first aspect of the invention.

Also disclosed is a gel obtainable by the method of the second aspect of the invention.

A third aspect of the invention provides a gel comprising two or more multicellular spheroids which are at predefined positions in a gel.

The multicellular spheroids formed by the methods of the invention, and therefore contained in the gels of the invention, are believed to have a nearly homogenous spherical shape, wherein the average diameter of the spheroids reaches from 50 to 2000 micrometer. preferably from 150 to 1000 micrometer and most preferably from 120 to 500 micrometer or 200 to 500 micrometer, such as from 250 to 350 micrometer. Thus, in one embodiment, the gel comprises one or more multicellular spheroids that have a homogeneous spherical shape with an average diameter from 50 to 2000 micrometer.

The multicellular spheroids formed by the methods of the invention, and therefore contained in the gels of the invention, may also be characterised in that they exhibit characteristics that substantially mimic those of the tissue of origin. Thus, at least one of the antigen profile, genetic profile, tumour biology, tumour architecture, cell proliferation rate(s), tumour microenvironment, therapeutic resistance, cell composition, gas concentrations, cytokine expression, growth factor expression and cell adhesion profile of the one or more multicellular spheroids may be substantially identical to that of the tissue of origin.

Accordingly, the multicellular spheroids exhibit a substantially similar or identical behaviour to that of natural cell systems, for example with respect to organisation, growth, viability, cell survival, cell death, metabolic and mitochondrial status, oxidative stress and radiation response as well as drug response.

As mentioned above, injection of a cell suspension into a gel allows for the formation of spheroids at predetermined locations. Thus, the gel of the invention comprises two or more multicellular spheroids which are at predefined positions in the gel. Preferred positioning and spacing between the two or more multicellular spheroids are as defined above with respect to the first aspect of the invention. In a further embodiment, the two or more multicellular spheroids in the gel of the third aspect of the invention respectively have at least two different cell compositions. Suitable cell types include those mentioned above.

Accordingly, a fourth aspect of the invention provides a gel comprising at least two multicellular spheroids at predefined positions in the gel wherein at least two multicellular spheroids have different cell compositions.

As mentioned above, the multicellular spheroids produced by the methods of the invention exhibit a substantially similar or identical behaviour to that of natural cell systems, making them particularly useful for 3D cell assays.

A fifth aspect of the invention provides a method of assessing the property of a cell selected from any of survival, growth, proliferation, differentiation, migration, morphology, signalling, metabolic activity, gene expression and cell-cell interaction, the method comprising (i) producing a multicellular spheroid according to the first aspect of the invention or producing a gel according to the second aspect of the invention or providing a gel according to the third or fourth aspects of the invention, and (ii) assessing the property of the cell in the multicellular spheroid.

It will be appreciated that the method allows the assessment of how a particular cell type affects the function of another cell type, for example where different cell types are present in the same multicellular spheroid or where two or more multicellular spheroids with at least two different cellular compositions are in close proximity to each other.

Assessing one or more properties of a cell may be carried out using any suitable method known in the art, either from cell spheroids or from spheroids fixed by snap-freezing or chemical fixation techniques. For example, any of cell survival, growth, proliferation, differentiation, migration and morphology may be assessed by microscopy or image analysis, as described, for example, in Example 1. Properties may be detected using appropriate markers. For example, expression of detectably-labelled proteins, reporters and/or single-step labelling of cell components and markers can enable cell architecture, multicellular organisation and other readouts to be directly visualised, for example by fluorescence microscopy (e.g. E-cadherin staining to identify cell-cell contacts). Gene expression may be assessed by functional genomic (eg microarray) techniques, and so on. Any of immunofluoroscence, Hoeschst staining or Annexin-V assays may be used. It is appreciated that the skilled person may select the appropriate technique to assess a given property.

A sixth aspect of the invention provides a method of assessing the effect of an agent on the property of a cell selected from any of survival, growth, proliferation, differentiation, migration, morphology, signalling, metabolic activity, gene expression and cell-cell interaction, the method comprising (i) producing a multicellular spheroid according to the first aspect of the invention or producing a gel according to the second aspect of the invention or providing a gel according to the third or fourth aspects of the invention, and (ii) assessing the effect of the agent on the property of a cell in the multicellular spheroid.

It will be appreciated that the method allows the assessment of the effect of an agent on how a particular cell type affects the function of another cell type, for example where different cell types are present in the same multicellular spheroid or where two or more multicellular spheroids with at least two different cellular compositions are in close proximity to each other.

By agent, we include any of a polypeptide, a peptide, a nucleic acid, a small molecule, or a natural product. Thus, the agent may be a drug or a biologically active agent. The agent may be an inhibitor of a particular cellular function.

It will be appreciated that the method allows one to assess the function of a cellular gene or protein, for example by using an agent that is an inhibitor of that gene or protein, as described further in the Examples. For instance, the agent may be any of a chemical inhibitor, a peptide inhibitor, a siRNA molecule or a shRNA construct or any agent capable of effecting a gene knockdown. It may also be desirable to use more than one inhibitor (eg with different selectivities) to provide further insight into the function of a cellular gene or protein. Similarly, by exposing multiple multicellular spheroids to a range of respective agents (eg RNAi inhibitors), the methodology can be scaled up to investigate the cellular functions of genome or proteome arrays on a larger scale.

In one embodiment, the agent is an infectious agent such as a bacterium or virus. In this way, the method may be used to study infection related processes such as whether cells are infected by bacteria or viruses and, if so, how the infection progresses and spreads. It may also be desirable to include a further agent so as to assess the effect of the further agent on the infection.

In another embodiment, the agent is a further cell. Thus, cells may be seeded on the surface of a gel containing one or more multicellular spheroids, and the effect of the further cell on the cells within the multicellular spheroid assessed. For example, the multicellular spheroid may comprise macrophages and the agent comprises skin cells, or vice versa, such that the interaction between macrophages and skin cells can be studied.

The agent may be applied to the multicellular spheroid after formation, or may be present in the gel in which the multicellular spheroid is formed, or it may be injected along with the cell suspension. In one embodiment, the agent may be contained within a bead so as to control the rate at which the agent is released (e.g. slow-release). Alternatively, the agent may be one that is expressed in the cells of the multicellular spheroid, such as a polynucleotide. A particular example is the screening of cDNA libraries or siRNA libraries, for example to screen for genes involved in particular signalling pathways.

It is appreciated that the method includes identifying an agent that modulates one or more properties of a cell selected from any of survival, growth, proliferation, differentiation, migration, morphology, signalling, metabolic activity, gene expression and cell-cell interaction, the method comprising (i) producing a multicellular spheroid according to the first aspect of the invention or producing a gel according to the second aspect of the invention or providing a gel according to the third or fourth aspects of the invention, and (ii) assessing the effect of the agent on the property of a cell in the multicellular spheroid.

In a particular embodiment of the above aspect of the invention the agent is a drug-like compound or lead compound for the development of a drug-like compound.

The term "drug-like compound" is well known to those skilled in the art, and may include the meaning of a compound that has characteristics that may make it suitable for use in medicine, for example as the active ingredient in a medicament. Thus, for example, a drug-like compound may be a molecule that may be synthesised by the techniques of organic chemistry, less preferably by techniques of molecular biology or biochemistry, and is preferably a small molecule, which may be of less than 5000 daltons and which may be water-soluble. A drug-like compound may additionally exhibit features of selective interaction with a particular protein or proteins and be bioavailable and/or able to penetrate target cellular membranes or the blood:brain barrier, but it will be appreciated that these features are not essential.

The term "lead compound" is similarly well known to those skilled in the art, and may include the meaning that the compound, whilst not itself suitable for use as a drug (for example because it is only weakly potent against its intended target, non-selective in its action, unstable, poorly soluble, difficult to synthesise or has poor bioavailability) may provide a starting-point for the design of other compounds that may have more desirable characteristics.

Thus in one embodiment, the method further comprises modifying an agent which has been shown to modulate at least one of the properties listed above, and testing the ability of the modified agent to modulate at least one of the properties listed above.

As well as being useful in drug development, the method of the sixth aspect of the invention may also be useful in personalised medicine regimes. For example, the method may be used to test the safety or efficacy of potential drug treatments, and so may aid the customisation of treatments for individual patients.

It is appreciated that assays which are capable of high throughput operation are particularly preferred. Thus, the assays may be performed on gels comprising multiple multicellular spheroids on multi-well plates as described above.

Further, the assays are preferably automated or semi-automated. As explained above, one advantage of the present method to produce multicellular spheroids is that the spheroids are formed at predefined locations. Thus, automatic detection of cell properties, for example, by automated microscopy, is much easier. Thus, in one embodiment, the assessment of the one or more of the cell properties listed above is automated.

Similarly, it is appreciated that, when the agent in the sixth aspect of the invention is applied to the gel after spheroid formation, application of the agent to the gel may be automated. Alternatively, where the agent in the sixth aspect of the invention is present in the gel prior to multicellular spheroid formation, the formation of the multicellular spheroids in the gel may be automated.

It is appreciated that the methods of the fifth and sixth aspects of the invention may be performed on pre-made gels according to the third or fourth aspects of the invention. Alternatively, the methods may involve first producing the multicellular spheroids according to the method of the first aspect of the invention, which is conveniently automated or semi-automated.

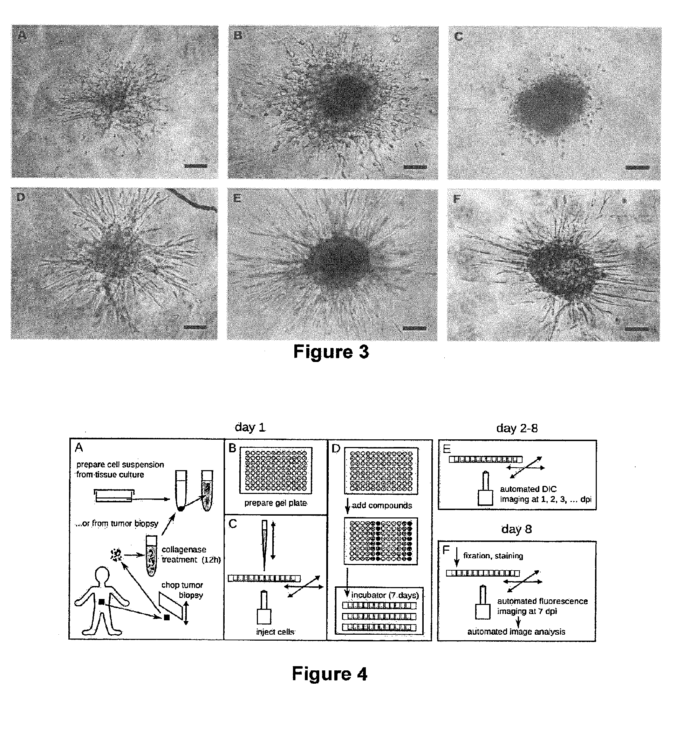

Figure 4 provides a schematic overview of various steps in the method of the sixth aspect of the invention that may be automated.

Preferences for the cells assessed in the fifth or sixth aspects of the invention include all of those mentioned above with respect to the first aspect of the invention. In a preferred embodiment, the cell is a cancer cell. Thus, the methods may be used to assess various properties of cancer cells or to determine the effects of particular agents (e.g. candidate drugs) on, for example, cancer cell invasion or migration. However, it is appreciated that the properties of other cell types may also be assessed including stem cells, endothelial cells and immune cells and that the methods have equal application in any one or more of stem cell biology, angiogenesis, immune biology, toxicity studies and tissue engineering. For example, it is within the scope of the invention to rebuild a metastatic microtumour e.g., tumour cells with hepatocytes, or tumour cells with bone marrow cells.

The invention provides the use of a gel according to the third or fourth aspects of the invention in assessing the property of a cell selected from any of survival, growth, proliferation, differentiation, migration, morphology, signalling, metabolic activity, gene expression and cell-cell interaction, or in assessing the effect of an agent on the property of a cell selected from any of survival, growth, proliferation, differentiation, migration, morphology, signalling, metabolic activity, gene expression and cell-cell interaction; or in tissue engineering.

For the avoidance of doubt, multicellular spheroids and gels of the invention may be used for research, diagnostic and/or therapeutic purposes, for example pharmacokinetic profiling, pharmacodynamic profiling, efficacy studies, cytotoxicity studies, penetration studies of compounds, therapeutic resistance studies, antibody generation, personalised or tailored therapies, RNA/DNA 'drug' testing, small molecule identification and/or testing, biomarker identification, tumour profiling, hyperthermia studies, radioresistance studies, tissue engineering and the like.

In a particular embodiment, the methods of producing multicellular spheroids according to the invention are used in tissue engineering.

For example, tissues can be produced by injecting cells into a gel scaffold. This has two main advantages compared to conventional scaffold-based tissue engineering approaches. Firstly, cells are directly placed together such that cell-cell contacts form rapidly. Secondly, different types of cells can be placed at predefined spots with higher precision as the cells are applied locally as opposed to flushed over a large scaffold surface. Thus, the method is particularly suitable for tissues which feature multiple cell types art close proximity.

Another application of the method in tissue engineering is in organ printing. This involves the layer-by-layer robotic biofabrication of three-dimensional functional living microtissues and organ constructs as described in Mironov et al (Biomaterials 30: 2164-2174, 2009) and in Moon et al (Tissue Engineering 16(1): 157, 2010). Using the method of producing multicellular spheroids of the invention, a gel rod may be pre-seeded with a high density concentration of cells to form an array of multicellular spheroids. Preferably, the spheroids are less than 300 micrometer in diameter so as to prevent necrosis, and preferably, the diameter of the rod is of the same order of magnitude as the spheroids. The gel rod may then be used to dispense cells in a controlled manner so as to print three-dimensional tissue structures (see Mironov *et al* and Moon *et al*). This has the advantage that cell-cell contacts form immediately, and start migrating and/or forming tissue after printing. Alternatively, the method of producing multicellular spheroids of the invention may be used to form multicellular spheroids at defined positions in a gel so as to print a three-dimensional tissue structure in the gel directly. Preferably, the spheroids are less than 300 micrometer in diameter so as to prevent necrosis. Also, to enhance nutrition delivery to the cells/multicellular spheroids, the gel may contain channels or pores, or volumes of unstable gel/sacrificial material which at a later stage form such channels or pores, in order to enable perfusion of nutrient-rich medium or blood. It will be appreciated that the combination of known bioprinting techniques with the injection method of the invention may also be desirable. For example, the method of the invention may be used to place specific cells only where they are needed once a three-dimensional structure has been formed (e.g. by printing or seeding a scaffold).

Also disclosed is a kit of parts comprising a gel, an injector, and a cell suspension.

The kit of parts may further comprise a means for assessing the property of a cell selected from any of survival, growth, proliferation, differentiation, migration, morphology, signalling, metabolic activity, gene expression and cell-cell interaction

Preferences for the gel, injector and cell suspension include those described above with respect to the first aspect of the invention.

The invention will now be described with the aid of the following Figures and Examples.
**Figure 1****:** Bright-field images showing spheroid formation process in 4T1 cells. A,B,C demonstrate hanging drop method on day 1,2, and 3. D,E,F show injection method of creation spheroids. G shows first migration form hanging drop spheroid on day 4, H shows similar migration from injected spheroid on day 1 (all scales = 120µm). E-cadherin staining in I, J show establishment of cell-cell contacts between day 0 and day 3.

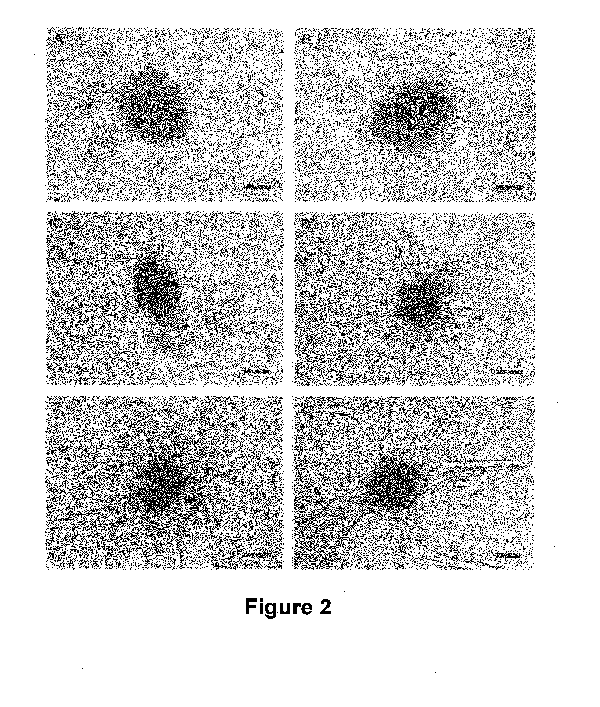

**Figure 2****:** Brightfield images showing the effect of high (2 mg/ml, left column) and low (1 mg/ml, right column) collagen concentration on spheroid invasion in breast cancer cells. First row(A,B): MTLn3-rat, second row (C,D): MDA-MB-231- human cell line , and third row (E,F): 4T1-mouse. Scale-bar represents 120 um.
**Figure 3****:** Brightfield images showing migrating spheroids of different cell types after 3 days. Top row shows single cell migration, in (A) HT1080 (collagen: 2mg/ml), in (B) MDA MB-231 and (C) MTLn3, then the second row shows collective migration, tumor cells in (D) 4T1 (collagen: 2mg/ml), and (E) PC-3 and normal HMEC cells in (F). Scale bar represents 120 um.
**Figure 4****:** Schematic overview of high-throughput spheroid screening. Cells are collected from cell culture or from fresh tissue and mixed with PVP. (A) Collagen gel is dispensed into a 96-well plate; (B) and after solidification, the cell-PVP suspension is automatically injected into the gels, thereby forming the cell spheroids. (C); Compounds are dispensed on top of the gel in the wells, and the 96 well plate is placed in a cell incubator. (D); During culture, the cells can be imaged using DIC microscopy. (E) Finally, the cells are fixed, stained, and imaged using fluorescence microscopy and the results quantified using automated image analysis (F).
**Figure 5****:** Results of automated injection of 4T1 cells (murine breast carcinoma cells) in a 96 well plate. Flatbed scanner image in A shows a bottom view of injected spheroids (scale = 10mm). Stitched Brightfield images on row 2 show the spheroid migration process on day 0 (B), day 2(C) and day 4 (D), scale is 1 mm. Bottom row shows single bright-field images of a migrating spheroid taken from the row above at day 0(E), day 2(F) and day 4(G). Scale bar represents 100 um. In (5H) the spheroid size is visible, in wells measured over two diagonal lines over the 96 well plate. (5I) shows the relative increase in size per spheroid with respect to the diameter at day 0, measured after 4 days in the same set of wells.

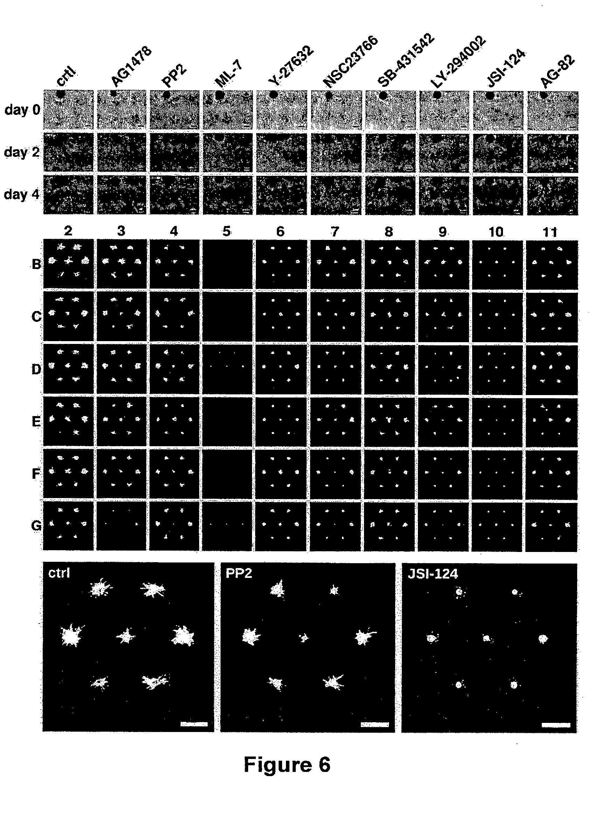

**Figure 6****:** Results from a drug screen performed on 4T1 cells in a 96 well plate. Columns show different compounds added to the wells containing the spheroids. Top: DIC images show tumor cell outgrowth in detail at day 0, day 2 and day 4 (scale = 100 µm). Cells are stained for actin and fluorescence is measured. After thresholding, the area of the spheroids is clearly visible in the whole plate (middle) and three wells are shown in more detail (crtl: 2D, PP2: 4D, JSI-124: 10D) on the bottom row.

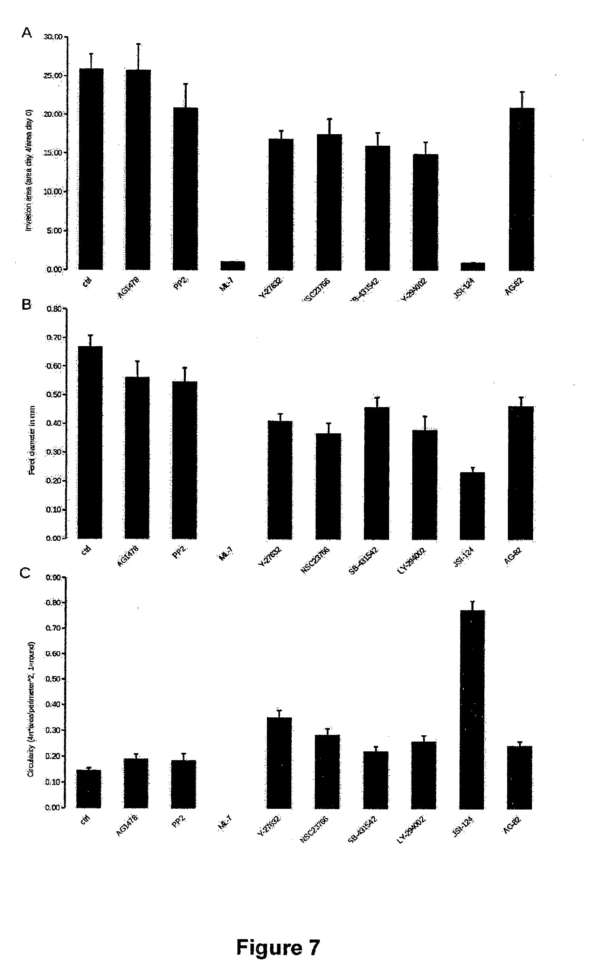

**Figure 7****:** Quantification of the spheroid measurements shown in Figure 6 of two rows (C,F, see Figure 6) containing the same concentration per compound. From the DIC images manually the feret diameter is determined at day 0 and day 4, and the relative increase is shown in A. Automated data analysis was performed on the fluorescently labeled spheroids resulting in the Feret diameter (B) and circularity (C). For each bar two wells containing a maximum of 14 spheroids were examined. From the fluorescent data it can be seen that cells treated with ML-7 did not survive the treatment.
**Figure 8****:** Spheroids obtained from fresh primary mouse cells, orthotopic tumors derived from 4T1 cells expressing a GFP marker. Six spheroids were created per well, an overview is visible in A (DIC), and B (fluorescence), scale represents 1 mm. The upper left spheroid (red square) is shown in more detail (GFP in C) after staining with mCherry-actin (D) and Hoegst (E). In F an overlay is visible showing co-localization of the GFP tumor cells with the actin staining (scales: 500µm).

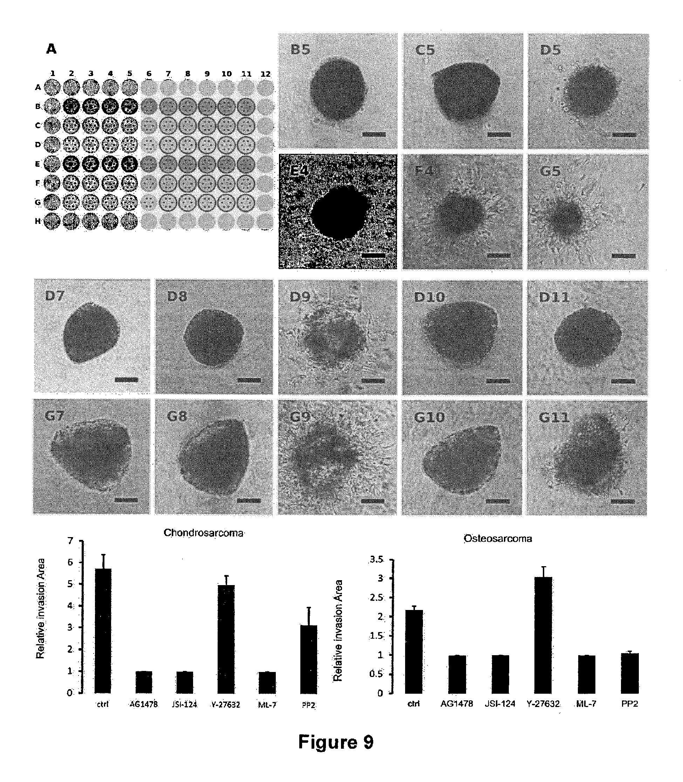

**Figure 9****:** Spheroids obtained from two fresh human biopsies. An overview of the 96 well plate is demonstrated in (A), in each well 6 spheroids were formed, the edge wells were not injected; three different collagen concentrations were used (row B,E 2.0; C,F 1.5; D,G 1.0 mg/ml) for Osteosarcoma in row B,C,D and Chondrosarcoma in row E,F,G. Encircled wells were treated with inhibitors: Columns 7: AG1478, 8: JSI-124, 9: Y-27632, 10: ML-7, 11:PP2 After 6 days bright-field images were obtained, and a representative set of single spheroids from different wells is visible: B5,C5,D5 and E4,F4,G5 respectively show the spheroids response to different collagen concentrations. From the control groups, see B5, C5 and D5 for the osteosarcoma results, the lowest concentration, row D, triggers individual cell migration (see D5) while higher concentrations shows almost no migration. The lowest concentration, row G, triggers the largest migration in Chondrosarcoma as well (G5) while the type of migration remains the same between the row F and row G (see F4, G5). Looking only at the lowest concentration of collagen it can be seen that four inhibitors block migration of Osteosarcoma (D7, D8, D10, D11) whereas the rock inhibitor stimulates migration as expected (D9). In Chondrosarcoma three inhibitors block migration completely (G7, G8, G10), one, PP2 reduces migration, similarly to screening results of breast cancer, mentioned earlier, and the rock inhibitor again stimulates migration as can been seen in G9. Scale = 100µm.

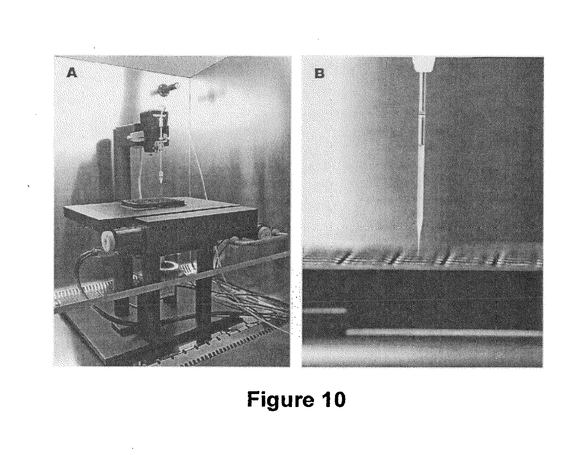

**Figure 10****:** The photo in (A) shows the injection robot, a frame holding a motorized stage, micromanipulator and camera with lighting. On the right side (B) a close-up of a filled injection needle above a well in a 96-well plate.

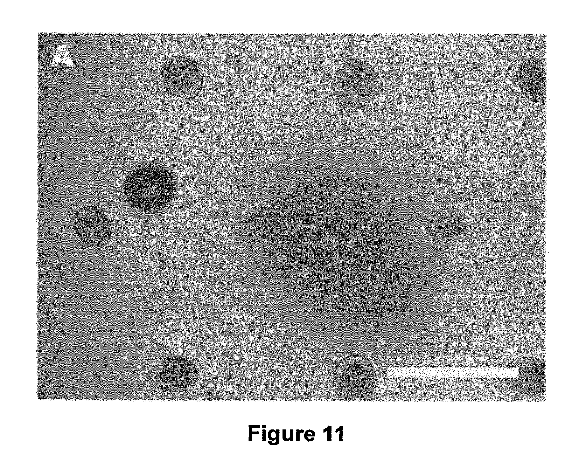

**Figure 11****:** Bright-field image of spheroids just after injection. Scale is 1 mm.

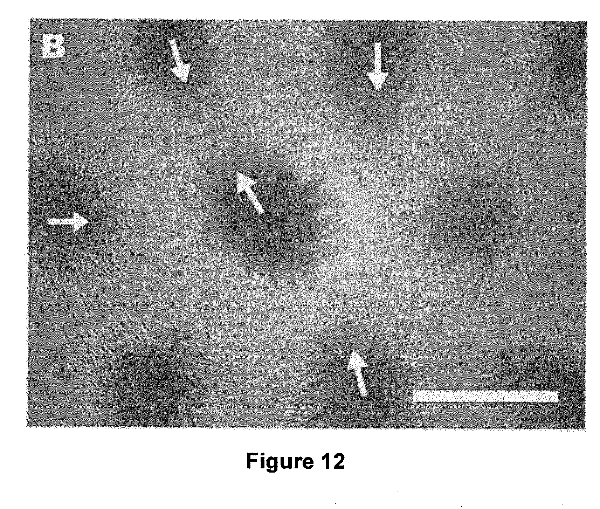

**Figure 12****:** Bright-field image 4 days after injection showing directional migration, indicated by arrows. Scale is 1 mm.

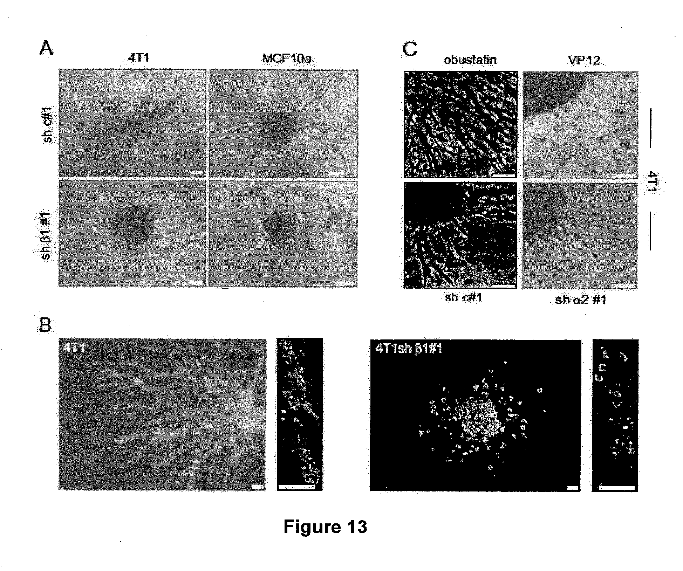

**Figure 13****:** Integrin control of 3D migration patterns. A, invasion of 4T1 and MCF10a expressing indicated shRNA constructs in collagen gels. B, invasion of 4T1 incubated with indicated peptides (top) or expressing indicated shRNA constructs (bottom) in collagen gels. C, E-cadherin staining of 4T1 and 4T1shβ1 in col1agen gels.

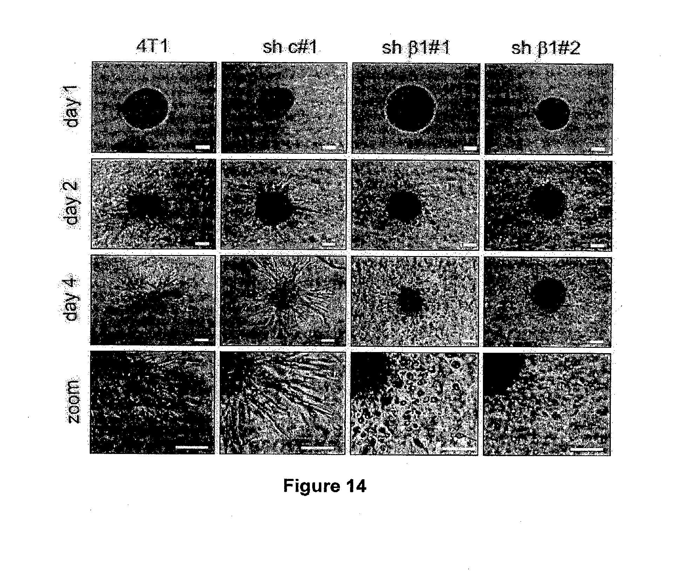

**Figure 14****:** 3D collagen invasion of 4T1 expressing indicated shRNA constructs at the indicated timepoints.

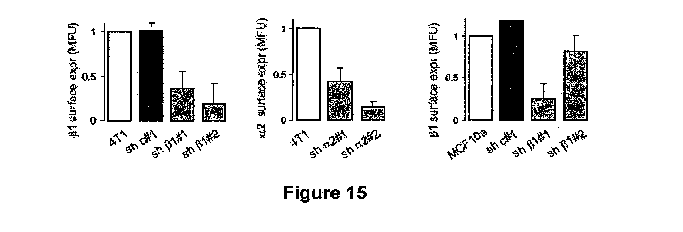

**Figure 15****:** FACS analysis of β1 or α2 integrin surface expression in 4T1 or MCF10a cells expressing indicated shRNA constructs.

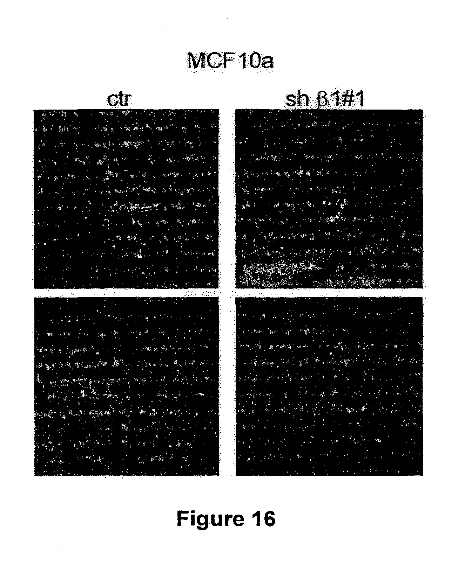

**Figure 16****:** Alexa-488-conjugated Annexin V labeling in MCF10a and MCF10a-shβ1 spheroids in collagen gels.

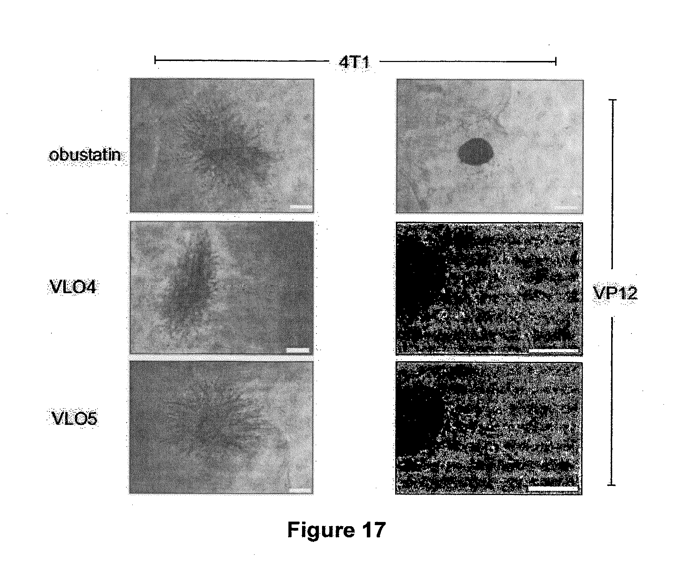

**Figure 17****:** Invasion pattern of 4T1 cells in collagen gels incubated with indicated peptides.

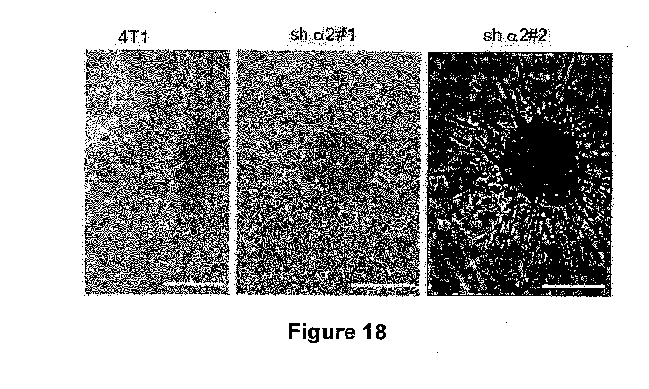

**Figure 18****:** 3D collagen invasion of 4T1 expressing indicated shRNA constructs.

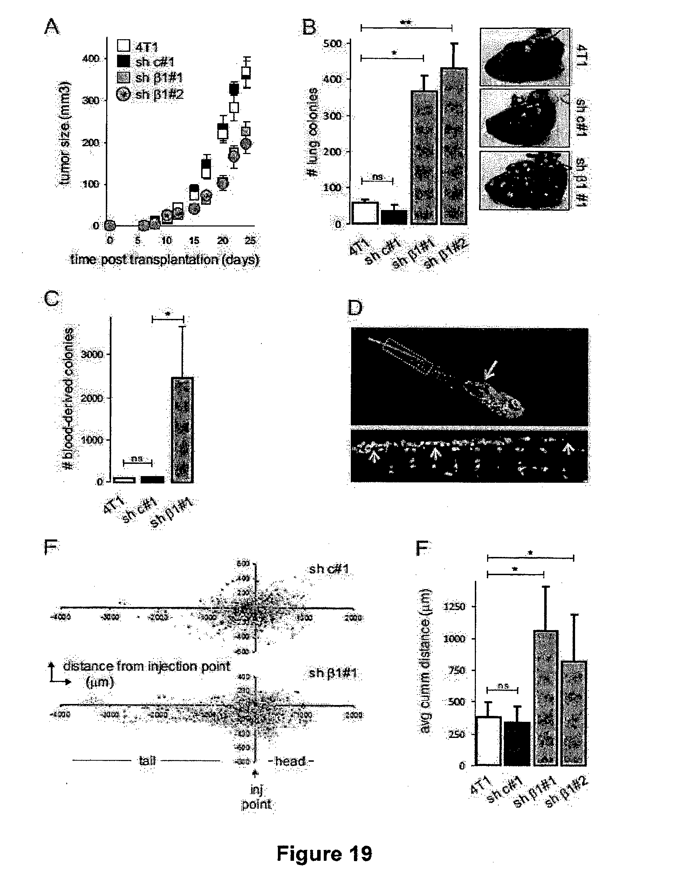

**Figure 19****:** Integrin control of *in vivo* migration. A-C, primary tumor growth (A), spontaneous metastasis (B), and circulating tumor cells (C) following orthotopic transplantation of 4T1 cells expressing indicated shRNA constructs in mammary fat pad. D, labeled 4T1shβ1 cells injected in zebrafish yolk sac (top) and spread towards tail (bottom). E, graphic representation of ~50 embryos from 2 independent experiments in which 4T1 cells expressing indicated shRNA constructs were injected. F, average cumulative migration distance calculated from E. *p<0.05; **p<0.001.

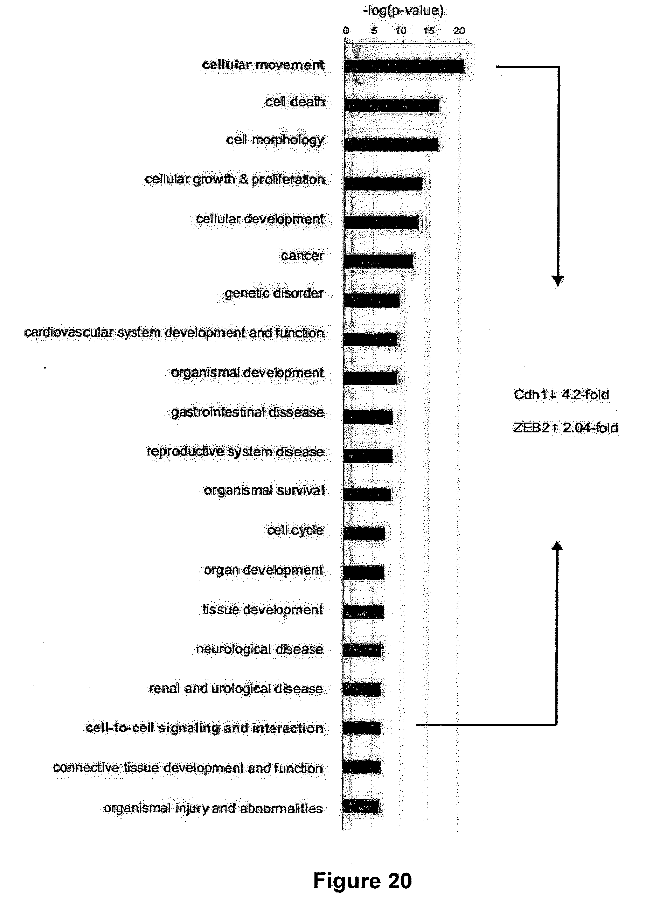

**Figure 20****:** Ingenuity Pathway Analysis ranking of processes affected specifically by two shβ1 constructs but not sh-c constructs, based on micro-array study. Fold change of E-cadherin and Zeb2 mRNA in shβ1 is indicated.

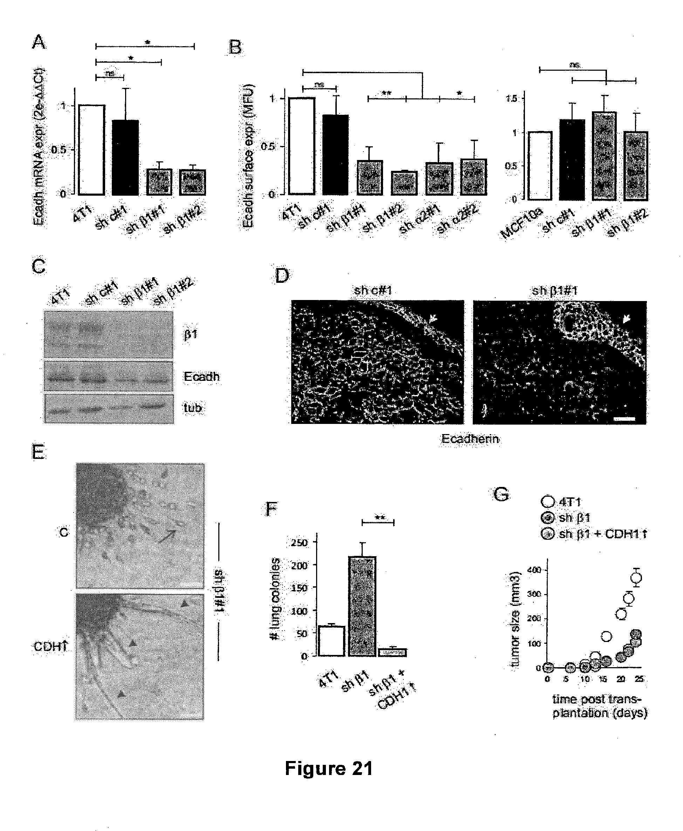

**Figure 21****:** E-cadherin expression mediates integrin regulation of invasion and metastasis. A-C, E-cadherin mRNA (A), surface expression (B), and total protein (C) levels in 4T1 or MCF10a cells expressing indicated shRNA constructs. (D), E-cadherin staining in 4T1 and 4T1shβ1 tumors. (E), invasion in collagen gels of 4T1shβ1 cells in absence (top) or presence (bottom) of E-cadherin cDNA. (F-G), Lung metastasis (F) and primary tumor growth (G) of orthotopically transplanted 4T1 cells expressing indicated shRNA and cDNA constructs. *p<0.05; **p<0.001.

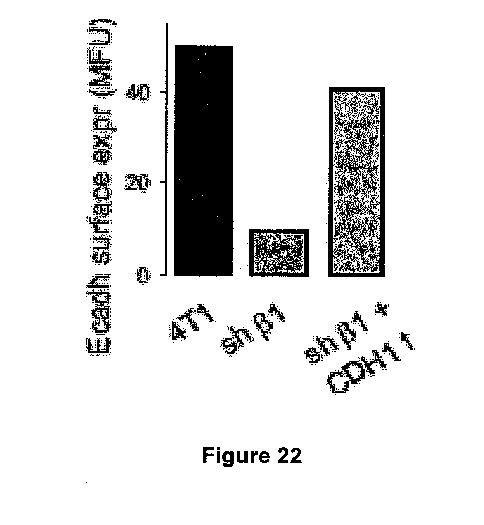

**Figure 22****:** FACS analysis of E-cadherin surface expression in 4T1 cells expressing indicated shRNA and cDNA constructs.

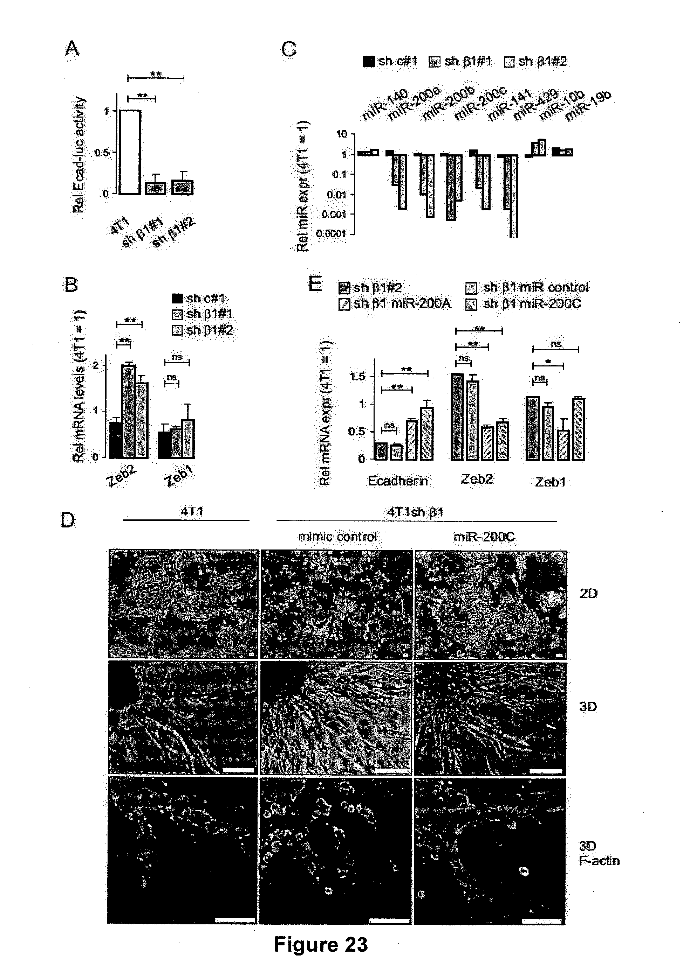

**Figure 23****:** Integrin regulation of miR-200/ZEB balance controls E-cadherin expression and cohesive migration. A-B, E-cadherin promoter activity (A) and Zeb1 and Zeb2 mRNA levels (B) in 4T1 cells expressing indicated shRNA constructs. C, E-cadherin surface expression in 4T1 transiently expressing indicated siRNAs. D, miRNA expression in 4T1 expressing indicated shRNA constructs. E, 4T1 cohesion suppressed by shβ1 and restored by synthetic expression of miR-200C in 2D culture (top) and in 3D collagen gels (middle and bottom). F, E-cadherin, Zeb2, and Zeb1 mRNA levels in 4T1shβ1 cells expressing indicated synthetic miR expression constructs. *p<0.05; **p<0.001.

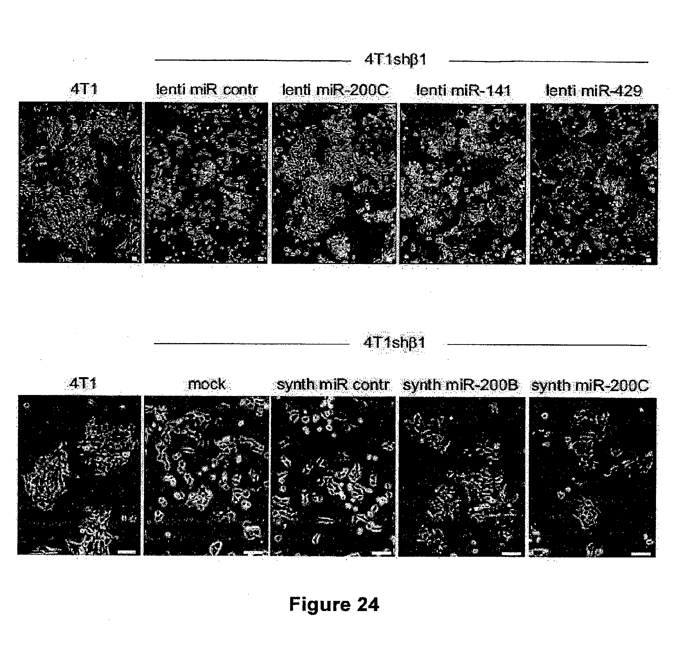

**Figure 24****:** 4T1 cohesion in 2D culture suppressed by shβ1 and restored by lentiviral (top) or synthetic (bottom) expression of indicated miR-200 species.

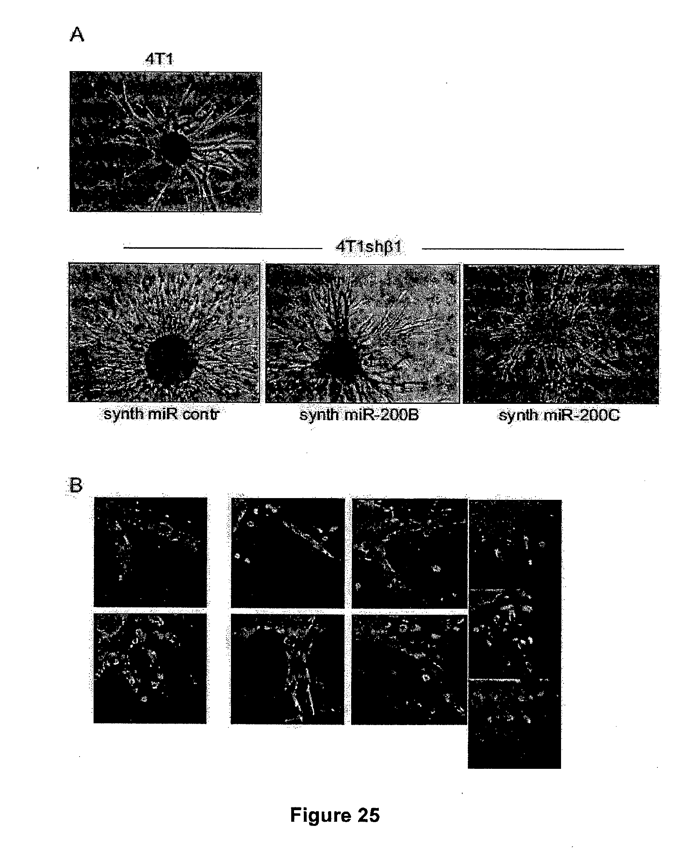

**Figure 25****:** 4T1 cohesion in collagen gels suppressed by shβ1 and restored by synthetic expression of indicated miR-200 species. DIC (top) and actin staining (Phalloidin; bottom) is shown.

### Example 1: Automated microinjection method generates multicellular spheroids for high throughput screening of cancer cell migration

### Summary

Multicellular spheroids (MS) are used to study cell behavior in a 3D extracellular environment that mimics the *in vivo* context more closely than standard 2D cell culture. Current methodologies do not allow MS formation with defined spatial distribution at high speed and high throughput. Here, we describe a novel method where cell-polymer suspensions are microinjected as droplets into collagen gels. MS formation time is strongly reduced compared to other methods and can be applied to a broad range of cell types including endothelial cells, various cancer cell lines, and primary tumor cell suspensions.

For high throughput screening purposes, we have automated this method to produce 7 MS per well with defined x-y-z spatial coordinates in 96 well plates and applied automated image analysis algorithms. Low intra- and inter-well variation of initial MS size, MS expansion, and cell migration of cells out of these MS indicates excellent reproducibility. We perform a proof-of-principle chemical screen on MS derived from breast cancer cells to identify compounds affecting cancer cell invasion/migration. Finally, we demonstrate applicability to freshly isolated tumor material from mouse and human biopsies and show the potential of this automated method to develop personalized cancer treatment strategies.

### Introduction

Cells grown under classical 2D culture conditions have been shown to behave differently from the same cell types grown *in vivo.* For instance, cell survival, proliferation, differentiation, cytoarchitecture, and migration can be altered on a 2D substrate (Kenny PA et al Molecular Oncology. 2007;1(1):84-96). Various systems have been developed to culture cells in 3D environments, aimed at mimicking the *in vivo* context. For instance, secretion of angiogenic factors and chemosensitivity of tumor cells in 3D cultures more closely resembles the *in vivo* situation as compared to 2D (Fischbach et al, 2007). Several of these systems produce 3D cell aggregates in which after compaction, depletion of oxygen, nutrients, and growth factors can occur in the core, leading to cell heterogeneity depending on the position in the resulting multicellular spheroid (MS; Mueller-Klieser, 1987; Sutherlands, 1988). Mostly, methods are used in which compact aggregates are formed first and subsequently placed within a 3D matrix. The best-known example of this approach is the hanging drop assay that was developed to create embryoid bodies from ES cells (Keller, 1995). In this method, spheroids are generated by using the natural disposition of cells to aggregate at the bottom of a droplet. This method has also been used for non-embryonic cell types, including cancer cells to produce tumor-like structures (Kelm et al, 2002). Alternative methods, involve mixing of a single cell suspension with a solidifying ECM, resulting in individual cells eventually forming spheroids randomly within a 3D ECM structure (Lee et al, 2007), or by seeding polymeric scaffolds with cell/ECM suspensions (Fischbach et al, 2007).

In 3D cultures, cell behavior, including proliferation and migration is strongly affected by chemical (composition) and physical (rigidity, cross-linking) properties of the gel. Natural ECM proteins can be used such as collagen, fibrinogen, or the laminin-rich matrigel to represent the *in vivo* ECM composition most relevant to a given cell type. More recently, synthetic polymers have been developed that can be used to support 3D MS culture environments (Loessner et al, 2010). Collagen type 1 is an abundant polymer in ECM *in vivo,* and it is widely used for 3D culture. It has been shown that various physical properties of the collagen gel, such as rigidity and pore size can have a major impact on stem cell differentiation, cancer growth, and cell migration (Buxboim and Discher, 2010; Friedl and Wolf, 2010; Leventhal et al, 2009). Cells can use various migration strategies in 3D environments, including mesenchymal or amoeboid individual cell migration modes or collective invasion strategies, depending on properties of the cells and of the matrix (Friedl and Wolf, 2010). Changes in matrix pore size can force cells to adopt alternative migration strategies or - if too extreme - pose a barrier to cell migration. Importantly, cells can modify the ECM by physical deformation and proteolysis, to overcome such barriers (Lammermann and Sixt 2009).

Chemical compound screens as well as RNAi screens for various types of cellular functions, including survival, growth, differentiation, and migration are mostly performed in 2D culture conditions. Current methods to analyze cells in 3D are labor and time intensive and may create a high level of variability between experiments. Most of these methods, such as the established hanging drop method, follow a three-step protocol of cellular aggregation, followed by a compaction phase, after which the resulting MS can be transferred into a gel. This limits their use to cell types that are cohesive and aggregate spontaneously. Moreover, variation in aggregation and compaction time creates a strong need for optimisation for each cell type and MS size is highly variable and the procedure as a whole is difficult and time consuming.

Alternative methods in which single cell suspensions are created in ECM substrates that are subsequently allowed to form a gel are relatively easy to perform but also have several major disadvantages: formation of MS depends on survival and proliferation of single cells in low adhesion conditions for extended periods; MS formation is time consuming; MS show a large variation in size; and MS form at random locations, which is disadvantageous for imaging purposes.

To allow for MS formation that is relatively fast and easy, highly reproducible, and that overcomes several of the disadvantages described above we have developed a novel method where cell-polymer suspensions are microinjected into multiwell plates containing a collagen gel. This method has been automated to produce MS with highly reproducible properties in large quantities in 96 well plates. As a proof of principle for applicability in high throughput screening efforts, we have used this system in a chemical screen for compounds that affect breast cancer invasion/migration. Finally, we show that this automated method can easily be applied to cell suspensions derived from fresh tumor biopsies. This provides the basis for testing therapeutic strategies on freshly isolated material of individual patients.

### Materials and Methods

### Cell culture

The following cell lines were obtained from ATCC: MDA-MB-231, MTLn3, PC-3, HT1080, 4T1, and MAE. GEβ1 was described earlier (Danen et al, 2002). All cell lines were cultured under standard cell culture conditions indicated by ATCC or described in (Danen et al, 2002) at 37°C, 5% CO₂ in a humidified incubator.

Primary mouse tumor cell suspensions were derived from surplus mouse breast tumor material by mincing using scalpel and tissue chopper followed by 2 hour collagenase treatment at 37°C.

Human biopsy material was obtained from surplus material from patients that were surgically treated for chondrosarcoma or osteosarcoma. Tumor cell suspensions were derived from biopsies by overnight collagenase treatment at room temperature.

### Preparation of collagen

Collagen type I solution was obtained from Upstate-Milipore or isolated from rat tail collagen by acid extraction as described previously [Rajan N, Habermehl J, Cote M, Doillon CJ, and Mantovani D. Nature Protocol 2007]. Collagen was diluted to indicated concentrations of ∼2.4 mg/ml in PBS containing 1xDMEM (stock 10x, Gibco), 44.04 mM NaHCO₃ (stock 440.4 mM, Merck), 0.1 M Hepes (stock 1 M, BioSolve).

### Hanging drop method

∼5,000 cells in 20 µl droplets were dispensed onto a 10 cm dish that was inverted over a dish containing 10 ml DMEM. After 24h, cell aggregates were harvested using a Pasteur pipette and transferred into 10 cm dishes coated with 0.75% agarose submerged in 10ml DMEM. After 48h spheroids had formed and these were embedded into a 2.4 mg/ml collagen solution using a Pasteur pipette. Collagen gels were allowed to solidify at 37°C for 30 min and overlaid with DMEM. Cell invasion was recorded for 3 days using an inverted phase contrast light microscope (Nikon Eclipse E600).

### Cell preparation for injection method

Cell suspensions derived from trypsin-detached adherent cultures or from collagenase-treated biopsies were filtered to remove clumps, centrifuged at 1000 rpm for 5 minutes, and washed twice with PBS. ∼7x10⁶ cells were re-suspended in 30 µl PBS containing 2% polyvinylpyrrolidone (PVP; Sigma-Aldrich). The PVP/cell suspension was loaded into a beveled pulled glass needle (Eppendorf CustomTip Type III, oD [µm] 60, Front surface 40, Flexibility: rigid).

### Manual injection

Cell suspensions in 2% PVP were microinjected (∼1x10⁴ cells/droplet) with a microinjector (20 psi, PV820 Pneumatic PicoPump, World Precision Instruments, Inc,) into solidified collagen gels in 8 well □̃slides (IBIDI).

### Automated injections

A glass-bottom 96 well plate (Greiner) containing 60 µl solidified collagen gel per well was placed in a motorized stage (MTmot 200x100 MR, Märzhäuser) connected to a controller (Tango, Märzhäuser). A motorized micro-manipulator (Injectman II, Eppendorf) was positioned above the stage and connected to a pump (Femtojet Express, Eppendorf) featuring an external compressor (lubricated compressor, model 3-4, JUN-AIR). A firewire camera (DFK41BF02.H, The Imaging Source) equipped with an 8x macro lens (MR8/O, The Imaging Source) was placed beneath the stage for calibration and imaging. All components were connected to the controlling computer (Ubuntu AMD64). A multi-threaded control program was written in Python using PySerial and wxPython. Coriander software (http://damien.douxchamps.net/ieee1394/coriander) was used for imaging.

After the program was calibrated for the 96 well plate the camera height was adjusted to focus on the bottom of the 96 well plate. The plate was then removed for needle calibration: the injection needle was fixed in the Injectman and moved, using the Injectman controller, into the center of the image. The injection height was set to 200 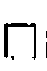 m above the bottom of the (virtual) plate. After the needle was moved up, the plate was placed back in position and the upper left well was used for multiple test injections to adjust pump pressure and injection time for optimization of the droplet size (300 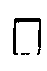 m diameter) using video inspection. Subsequently, using a pre-defined macro defining x-y coordinates and number of injections per well, all wells were injected using the same pressure and injection time.

### Microscopy and image analysis

Manually injected MS were monitored daily using a Nikon Eclipse E600 microscope. MS generated by automated injection were used for montage imaging using a Nikon TE2000 confocal microscope equipped with a Prior stage controlled by NIS Element Software and a temperature and CO₂-controlled incubator.

Differential interference contrast (DIC) images were captured using a charged coupled device (CCD) camera with NIS Image Pro software at 10x dry objective. Quantification of spheroid invasion area was analyzed from DIC images using ImageJ. The spheroid ellipsoidal area after three days was estimated using the diameter in x and y axis (pi*radius-x*radius-y) occupied by cells in the 10x montage image in the mid-plane of each spheroid and normalizing to the occupied area 1h after injection. One-way ANOVA was performed to test the significance of the data (p<0.05). The data are presented and plotted as average and standard error of the mean; tables are available in the supplement.

For automated imaging, wells containing gel-embedded spheroids were treated with a fixation and staining cocktail containing 3.7% formaldehyde, 0.2% Triton X-100 (Sigma) and 0.1 µM rhodamine phalloidin (Sigma) for 3 hrs. Wells were washed extensively with PBS and plates were imaged on a Becton Dickinson Pathway 855 using a 4X lens. A montage of 12 frames was made for each Z plane, with a total of 24 Z planes at an interval of 50 µm. Image stacks were converted into 2D maximum fluorescence intensity projections using ImagePro 7.0. MS were then digitally segmented using ImageJ to identify the outline of individual spheroids and multiple parameters were measured, including spheroid Feret's diameter, roundness, and number of spheroids scored in each well.

For immunostaining of E-cadherin, gels were incubated for 30 mins with 5 ug/ml collagenase (Clostridium histolyticum, Boehringer Mannheim) at room temperature, fixed with 4% paraformaldehyde, permeabilized in 0.2% Triton X-100, and blocked with 10% FBS. Gels were incubated with E-cadherin antibody (BD Transduction Laboratories) overnight at 4°C followed by Alexa 488-conjugated secondary antibody (Molecular Probes/Invitrogen) for 2 hrs at room temperature and Hoechst 33258 nuclear staining (Molecular Probes/Invitrogen) for 30 min at room temperature. Preparations were mounted in Aqua-Poly/Mount solution (Polysciences, Inc) and analyzed using a Nikon TE2000 confocal microscope. Z-stacks (~100 stacks, step of 1 µm) were obtained using a 20x dry objective, imported into ImageJ, and collapsed using extended depth of field plugin (Z projection) into a focused composite image.

### Drug Treatment

LY-294002 (phosphatidylinositol 3-kinase), JSI-124-cucurbitacin (STAT3/Jak2), and NSC23766 (Rac1) were purchased from Merck/CalBiochem. PP2 (Src) and ML-7 (MLC kinase) were purchased from ENZO. Y-27632 (Rock) and SB-431542 (TGFb) were purchased from BioMol Tocris. AG1478 (EGFR) was purchased from BioMol and AG-82 (general protein tyrosine kinases) was purchased from Calbiochem. Cell migration was analyzed in the absence and presence of inhibitors for 4 days.

### Results

To design a protocol that rapidly produces MS with highly reproducible characteristics we developed a novel method based on microinjection. For the microinjection method we mixed cells with polyvinylpyrrolidone (PVP), which is an inert (hydrophilic) water-soluble synthetic polymer, also used as emulsifier, food-additive (E1201) and as solubilising agent for injections (Haaf F, Sanner A and Straub F. 1985). In our application it was used to delay cell sedimentation within the capillary needle. Furthermore, it is believed that PVP traps the cells within the droplet allowing sufficient time for cell aggregation into a spheroid.

We first compared our method to the established hanging drop assay (Keller, 1995). 20 µl drops containing 5x10³ GEβ1 epithelial cells (Danen et al, 2002) were used to create hanging drops in an inverted 10 cm dish. The time required to form cell aggregates was 24h (Fig 1A). These cell aggregates were transferred to agarose-coated dishes where they formed tightly packed spheroids over a period of 48h (Fig 1 B). Next, the spheroids were embedded in 2.4 mg/ml collagen solution that was subsequently allowed to solidify (Fig 1 C). For microinjection, GEβ1 cells were suspended in 2% PVP and subsequently loaded into a pulled glass needle. Droplets of ~80 nl containing ∼1x10⁴ cells were injected directly into a preformed 2.4 mg/ml collagen gel where they formed tightly packed spheroids within 1 h (Fig 1D) and showed cells migrating out of these structures at later time points (Fig 1E,F). Comparing MS at 1 day post-injection to the hanging-drop-derived MS 1 day post placement in the collagen gel (4 days after initiating the hanging drop) showed that both methods displayed similar invasion patterns (Fig 1G,H). Using E-cadherin staining we observed that stable cell-cell contacts were established in 4T1 mouse breast carcinoma cells within one day (Fig I,J) and were maintained throughout the assay (not shown). Importantly, other cell types that are known to be non-cohesive (e.g. MDA-MB-231, which lacks E-cadherin) also readily formed MS (see below).

These results demonstrate that the microinjection method produces MS rapidly and conveniently compared to hanging drops (minutes versus days) while showing comparable migration/invasion behaviour.

Matrix rigidity has been shown to influence cell behavior in 3D (Lammermann and Sixt 2009). We studied how modification of the collagen concentration would affect MS formation and subsequent cell migration. For this purpose, a panel of mouse, rat, and human breast cancer cells 4T1, MTLn3, and MDA-MB-231 was microinjected into gels containing a range of collagen concentrations. All gels tested, similarly supported MS formation but subsequent cell migration from these MS was clearly affected by the collagen concentration in a cell type-dependent manner (Fig 2). At the highest collagen concentration migration of MDA-MB-231 and MTLn3 was hindered whereas 4T1 cells showed slow movement (Fig 2A,C,E). At the lowest collagen concentration all three cell lines migrated efficiently (Fig 2B,D,F).

Importantly, MDA-MB-231 readily formed MS whereas these cells have been reported to be unable to form compacted packed spheroids in hanging drop-, liquid overlay-, or other assays, without the need for additives such as matrigel (Andrea Ivascu and Manfred Kubbies, 2006).

We further analyzed migration of these and other cell types and identified several distinguishable migration strategies (Fig 3). HT1080 human fibrosarcoma and MDA-MB-231 human breast cancer cells, which do not typically form cell-cell contacts in 2D cell culture, invaded the collagen gel using a mesenchymal individual cell migration mode (Fig 3A,B). MTLN3 rat breast cancer cells adopted an amoeboid individual cell migration pattern (Fig 3C). Finally, 4T1 mouse breast cancer and PC-3 human prostate cancer cells as well as human microvascular endothelial cells (HMEC) that grow as islands in 2D culture, invaded as cohesive strands into the collagen matrix (Fig 3D,E,F).

These data indicate that the microinjection method rapidly produces MS from which different migration/invasion patterns can be analyzed for various cell lines including those that are incompatible with previous methods.

Since this novel method has the potential to rapidly create MS with high reproducibility for large-scale analysis of cell migration/invasion we set up a procedure to automate the spheroid formation process (Fig 4). For this purpose, a 96 well plate containing 60 µl collagen gel per well was placed on a motorized stage and the glass needle containing the cell/PVP suspension described above was placed vertically in a motorized micromanipulator above the stage (Fig 10). After calibration of both needle and 96-well plate using camera vision from under the stage, a computer script was used to automate the injection process with various macros.

With this set up, cell droplets were injected resulting in spheroids of ~300 µm diameter. To increase reproducibility, needle tip diameter variance was reduced by using commercial needles, and gels were prepared from a single large batch of collagen isolated in-house from rat tail. Various layouts of injection patterns were tested and we noted that a hexagonal pattern of 19 spheroids spaced at 1.2 mm showed interaction between spheroids (see arrows in Fig 12). Therefore, for screening purposes, we chose a less dense hexagon pattern of 7 spheroids where the spheroids were positioned 2 mm apart from each other, leaving enough space for outgrowth during the first 4 days (Fig 5A-G). Visual inspection indicated inhibition of outgrowth on the most outer rows and columns of each plate pointing to edge effects (not shown). We therefore chose to exclude these wells in all further experiments. To determine reproducibility in all other wells, we measured spheroid size throughout the plate and found that there was no significant intra- or inter-well variation (Fig 5H ANOVA, P=1.00). Moreover, outgrowth of MS over a 3-day time course was again not significantly different for different spheroids and different wells (Fig 5I ANOVA, P=0.797).

**Table of data belonging to Figure 5H**

| Spheroid area in mm^2 | | | | | | | | | | | | |
|---|---|---|---|---|---|---|---|---|---|---|---|---|
| | B4 | C5 | D6 | E7 | F8 | G9 | E2 | F3 | G4 | B9 | C10 | D11 |
| averages | 0.11 | 0.11 | 0.12 | 0.11 | 0.1 | 0.11 | 0.11 | 0.11 | 0.12 | 0.12 | 0.12 | 0.1 |
| std dev | 0.03 | 0.06 | 0.02 | 0.01 | 0.04 | 0.05 | 0.05 | 0.03 | 0.07 | 0.06 | 0.07 | 0.05 |
| # spheroids | 7 | 7 | 7 | 7 | 7 | 7 | 7 | 7 | 7 | 7 | 7 | 7 |
| std error | 0.01 | 0.02 | 0.01 | 0.01 | 0.02 | 0.02 | 0.02 | 0.01 | 0.03 | 0.02 | 0.03 | 0.02 |
| ANOVA-test | P = 1.00 | | | | | | | | | | | |

**Table of data belonging to Figure 5I**

| Invasion area (area day 4/ area day 0) | | | | | | | | | | | | |
|---|---|---|---|---|---|---|---|---|---|---|---|---|
| | B4 | C5 | D6 | E7 | F8 | G9 | E2 | F3 | G4 | B9 | C10 | D11 |
| averages | 6.16 | 5.85 | 5.43 | 6.17 | 6.25 | 7.09 | 6.13 | 7.16 | 5.97 | 7.37 | 5.75 | 8.95 |
| std dev | 2.11 | 4.34 | 1.76 | 3.79 | 2.44 | 3.37 | 1.55 | 1.14 | 0.92 | 2.27 | 2.64 | 4.31 |
| # spheroids | 7 | 7 | 7 | 7 | 7 | 7 | 7 | 7 | 7 | 7 | 7 | 7 |
| std error | 0.86 | 1.77 | 1.76 | 1.55 | 1 | 1.38 | 0.69 | 0.46 | 0.38 | 0.92 | 1.32 | 1.63 |
| ANOVA-test | P = 0.797 | | | | | | | | | | | |

This demonstrates that the microinjection method can be automated, which allows for up to 7 MS per well in 96 well plates, with precise determination of MS localization in x-y-z directions. Such properties make this protocol highly applicable to automated imaging strategies.

A proof of principle drug screen was performed to test the applicability of this procedure to automated high-throughput drug screening assays (HTS). 4T1 MS were generated, and various previously described compounds affecting cell migration and/or survival (AG1478, PP2, ML-7, Y-27632, NSC23766, SB-431542, AG-82, LY-294002, JSI-124) were added one hour later at different concentrations (4,10,20 µM) in duplicate. Effects on cell migration could be clearly observed by visual inspection after 4 days, especially for ML-7 and JSI-124 (Fig 6). We used rhodamine phalloidin to fluorescently label the actin cytoskeleton of cells within the spheroids. Analysis of these images indicated that ML-7JSI-124 affected cell survival whereas ML-7JSI-124 blocked migration, but left the MS intact (Fig 6). For one concentration (10 µM), all MS were quantified. Manual assessment of the Feret diameter from the DIC images at days 0 and 4 demonstrated that initial MS size, MS expansion, and effects of inhibitors were highly reproducible (Fig 7A). For automated imaging, we used the fluorescent actin stainings after thresholding. This allowed automated particle analysis and provided Feret diameter and circularity at day 4 (Fig 7B,C). The staining procedure is not compatible with analysis of spheroid dynamics as it is by definition an endpoint measurement. Nevertheless, the reproducibility of the injection procedure as shown in Figure 5, combined with the similarity between the analysis shown in Figures 7A and B, demonstrates that automated fluorescent imaging combined with automated particle analysis can provide a means to derive a fully automated methodology that is accurate and reproducible.

**Table of data belonging to Figure 7A**

| Values for 1:1000 dilution , Invasion area (area day 4/ area day 0) | | | | | | | | | | |
|---|---|---|---|---|---|---|---|---|---|---|
| | ctrl | AG14 78 | PP2 | ML-7 | Y-27632 | NSC237 66 | SB-431542 | LY-294002 | JSI-124 | AG-82 |
| average s | 25.88 | 25.75 | 22.4 8 | 1.09 | 16.86 | 17.51 | 16 | 14.91 | 1.01 | 20.9 6 |
| std dev | 6.96 | 12.04 | 9.90 | 0.10 | 3.80 | 7.10 | 6.15 | 5.68 | 0.08 | 7.40 |
| # spheroid s | 14 | 14 | 13 | 14 | 14 | 14 | 14 | 14 | 14 | 14 |
| std error | 1.86 | 3.22 | 2.74 | 0.03 | 1.01 | 1.90 | 1.64 | 1.52 | 0.02 | 1.98 |
| 2-tailed T-test | NA | 0.97 | 0.31 | 3.96 E-13 | 2.40E-4 | 4.07E-3 | 4.93E-4 | 1.05E-4 | 3.70E -13 | 8.14 E-2 |

**Table of data belonging to Figure 7B**

| Values for 1:1000 dilution, Feret's Diameter - The longest distance between any two points along the selection boundary, also known as maximum caliper | | | | | | | | | | |
|---|---|---|---|---|---|---|---|---|---|---|
| | ctrl | AG147 8 | PP 2 | ML-7 | Y-27632 | NSC237 66 | SB-431542 | LY-294002 | JSI-124 | AG-82 |
| averages | 0.6 7 | 0.56 | 0.5 5 | NA | 0.41 | 0.37 | 0.46 | 0.38 | 0.23 | 0.46 |
| std dev | 0.1 5 | 0.21 | 0.1 7 | NA | 0.09 | 0.14 | 0.13 | 0.17 | 0.07 | 0.12 |
| # spheroids | 14 | 14 | 13 | 0 | 14 | 14 | 14 | 13 | 14 | 14 |
| std error mean | 0.0 4 | 0.06 | 0.0 5 | NA | 0.02 | 0.04 | 0.03 | 0.05 | 0.02 | 0.03 |
| 2-tailed T-test | NA | 0.04 | 0.0 1 | NA | 2.08E-009 | 1.63E-008 | 3.81E-006 | 2.03E-006 | 1.58E-014 | 1.34E-005 |

**Table of data belonging to Figure 7C**

| Values for 1:1000 dilution, Circularity (4π*area/perimeter^2, 1=perfect circle) | | | | | | | | | | |
|---|---|---|---|---|---|---|---|---|---|---|
| | ctrl | AG147 8 | PP 2 | ML-7 | Y-27632 | NSC237 66 | SB-431542 | LY-294002 | JSI-124 | AG-82 |
| Averages | 0.1 5 | 0.19 | 0.1 9 | NA | 0.35 | 0.29 | 0.22 | 0.26 | 0.78 | 0.24 |
| std dev | 0.0 3 | 0.07 | 0.0 9 | NA | 0.11 | 0.08 | 0.07 | 0.09 | 0.13 | 0.06 |
| # spheroids | 14 | 14 | 13 | 0 | 14 | 14 | 14 | 13 | 14 | 14 |
| std error mean | 0.0 1 | 0.02 | 0.0 3 | NA | 0.03 | 0.02 | 0.02 | 0.03 | 0.03 | 0.02 |
| 2-tailed T-test | NA | 0.05 | 0.1 5 | NA | 2.28E-007 | 3.43E-006 | 1.05E-003 | 2.81E-004 | 5.93E-016 | 2.27E-005 |

Lastly, we tested the methodology with drug screening directly on freshly isolated primary tumor material. For this, cell suspensions were generated from orthotopic breast tumors in mice derived from 4T1 cells expressing a GFP marker. Importantly, in contrast to past biopsy spheroid methods our method circumvents any tissue culture steps, which may cause altered cell behavior (Bjerkvig 1990; Bissell 1981; Wapita and Hay 2002; Corcoran et al 2003; Beliveau et al, 2010). Following injection these rapidly formed MS from which migration was analyzed after 3 days (Fig 8A). To verify that surviving and migrating cells in these MS were tumor cells and not tumor-associated fibroblasts, MS were stained for actin and Hoechst. Indeed, the near complete overlap between actin and GFP staining demonstrated that these MS consist mainly of tumor cells (Fig 8B).

Next, cell suspensions were derived from freshly isolated human biopsies of osteosarcoma and chondrosarcoma and injected in gels containing three different collagen concentrations. MS readily formed from these human biopsies and survival and migration could be studied for up to 1 week with the two tumor types showing distinct migratory behavior (Fig 9). Osteosarcoma displayed a mixture of individual amoeboid and mesenchymal movement (Fig 9D5) whereas chondrosarcoma migrated as individual mesenchymal cells only (Fig 9G5). The 2 cell types showed different migratory responses to the different collagen concentrations but both migrated efficiently at the lowest concentration (Fig 9 B5-D5; E4-G5). MS were treated with the range of compounds described above starting 1 day post-injection. Several of the chemical inhibitors effectively inhibited migration of both tumor types, including AG1478, JSI-124, ML-7 and PP2 (Fig 9 D7-G11). Thus, the automated MS injection methodology has the potential to be used for large scale screening for the drug sensitivity spectrum of tumor cells freshly isolated from individual patients.

### Discussion

Here, we describe a method for generation of 3D MS cultures based on microinjection of cell suspensions into premade gels, that has a number of features making it highly useful for drug screening applications: compared to previous methods, it is easy (a one step procedure) and fast (minutes instead of days); MS are generated with high accuracy at predetermined x-y-z positions in multiwell plates; it is applicable to many different cell types irrespective of the ability of cells to form spontaneous cell-cell contacts (hence, it can be used for cell types such as MDA-MB-231 that will not compact spheroids by earlier described methods (Ivascu and Kubies, 2006, 2007); it shows good intra- and inter-well reproducibility with respect to MS size and outgrowth; and because of the pre-defined coordinates of each individual MS the method can easily be combined with fully automated imaging and image analysis protocols.

2D culture conditions are a very poor representation of the environment cells encounter *in vivo.* Besides implications for cell biology studies, this has important consequences for the interpretation of genetic - and drug screens ( Pampaloni F et al, 2007). So far, these have mostly been performed on 2D cultures. Various 3D culture systems have been developed but these have not been used for large-scale drug screens. This is due to the complicated procedures for generating 3D cultures, which negatively affect reproducibility of results and lead to higher costs. A number of 3D culture systems have developed. For the study of tumor cell invasion the Boyden chamber assay (trans-well migration assay) is most commonly used. Here, a monolayer of cells migrates through a thin layer of gel to reach the bottom of a filter. This particular assay does not resemble cells disassociating from a solid tumor. For this purpose, MS cultures have been developed that provide a pathophysiological context that mimics solid cancer microenvironments. Typically, MS can be derived from two methods: a complex multistep procedure in which spontaneous cell aggregates are transferred into a gel after compaction (e.g. the hanging-drop assay) or a single cell suspension is mixed with a solidifying gel and single cells grow out to form MS.

Our current method, for the first time provides a simple, one step procedure resulting in highly reproducible MS at defined coordinates. Reproducibility of MS size is critical for a reliable 3D culture platform. Size and compactness of MS will inevitably affect drug penetration and previous studies have indicated that MS with diameters between 200 and 500µm are required to develop chemical gradients (e.g. of oxygen, nutrients, and catabolites) that may represent conditions found in tumors (Friedrich et al., 2007a; Kunz-Schughart et al., 2004; Mueller-Klieser, 1987, 1997, 2000; Sutherland, 1988). Our automated approach yields spheroids with a diameter of ∼300µm, a size that best represents solid tumor traits.

We have used collagen based gels but the same method can easily be adapted to studies using alternative 3D matrices. We show that changing collagen concentrations impacts on cell migration and that optimal conditions differ for distinct cell types, in agreement with findings from others ( Bott et al, 2010; Lossner 2010; Sung et al,. 2009/ Biomaterials 30 (2009) 4833-4841). Hence, gel formation should be standardized and optimized for each cell type.

The use of ECM proteins such as collagen has some limitation in terms of being able to control batch-to-batch variation. Therefore, chemical crosslinking stabilization may be applied to control the mechanical properties (porosity and mechanical strength), which differ from batch to batch. A number of different cross-linking agents that react with specific amino acid residues on the collagen molecule, synthetic biopolymer scaffolds, and self-assembling synthetic oligopeptides gel are available to address this problem.(RossoF, Marino et al., 2005; Peppas et al, 2006; Sung et al, 2010 Pampaloni F et al 2007, Silver et al., 1995).

We demonstrate that we can automate each step of the procedure, from injection of cell suspension to imaging and image analysis, while maintaining reproducibility. Our method not only accelerates and simplifies MS formation but by generating up to 7 MS per well at predefined x-y-z coordinates it is compatible with fully automated imaging procedures, enhanced data collection, and robust statistical analysis. We present a small drug screen to demonstrate such properties.

Finally, we show that the method presented here can be used for MS formation directly from freshly isolated tumor biopsy material without the need of any intermediate culture steps. The exclusion of the intermediate step eliminates artificial traits found in 2D culture, and MS retain most of it *in vivo* properties. This opens the door to relatively high throughput screening on a patient-by-patient basis for drug sensitivity of tumor cells under conditions that may closely mimic the in vivo pathophysiological situation. Moreover, various expansions of this method can be envisioned in which multiple cell types are combined (e.g. cancer cells and cancer-associated fibroblasts and/or endothelial cells) to further improve representation of the complex tumor microenvironment that will ultimately affects tumor progression and drug sensitivity.

### Conclusions

MS derived by this novel microinjection method show similar growth and migration as spheroids derived from the classical hanging drop method. After microinjection, the cells are densely packed, allowing cell-cell and cell-matrix contacts to be formed almost immediately. Extensive testing using multiple different cell types reveals that MS can be formed using human, rat and mouse, cancer and non- cancer cell types including cell types which lack the ability to form cell-cell contacts are not compatible with previous methods.

Compared to previous methods there is a vast reduction in methodology steps and time required for MS formation. X-y-z coordinates can be predetermined, which strongly facilitates automated imaging procedures. As a result, formation of MS with high speed, high reproducibility, and high accuracy of positioning make our novel method highly suited to High Throughput Screening (HTS) applications.

Finally, this method allows the creation of *in vitro* tumor spheroids from fresh biopsy material without intermediate culture steps, thus providing an opportunity for screening biopsy material to customize treatment for individual patients.

### References

1. Lee GY, Kenny PA, Lee EH, Bissell MJ. Three-dimensional culture models of normal and malignant breast epithelial cells. Nat Methods 2007;4:359-365
2. Fischbach C, Chen R, Matsumoto T, Schmelzle T, Brugge JS, Polverini PJ et al. Engineering tumors with 3D scaffolds. Nat Methods 2007;4:855-860
3. Mueller-Klieser W. Multicellular spheroids. A review on cellular aggregates in cancer research. J Cancer Res Clin Oncol 1987;113:101-122
4. Sutherland RM. Cell and environment interactions in tumor microregions: the multicell spheroid model. Science 1988;240:177-184
5. Keller GM. In vitro differentiation of embryonic stem cells. Curr Opin Cell Biol 1995;7:862-869
6. Kelm JM, Timmins NE, Brown CJ, Fussenegger M and Nielsen LK. Method for generation of homogeneous multicellular tumor spheroids applicable to a wide variety of cell types. Biotechnol Bioeng 2003;83:173-180
7. Loessner D, Stok KS, Lutolf MP, Hutmacher DW, Clements JA and Rizzi SC. Bioengineered 3D platform to explore cell-ECM interactions and drug resistance of epithelial ovarian cancer cells. Biomaterials 2010;31:8494-8506
8. Buxboim A, Discher DE. Stem cells feel the difference. Nat Methods 2010;7:695-697
9. Friedl P, Wolf K. Plasticity of cell migration: a multiscale tuning model. J Cell Biol 2010;188:11-19
10. Levental KR, Yu H, Kass L, Lakins JN, Egeblad M, Erler JT et al. Matrix crosslinking forces tumor progression by enhancing integrin signaling. Cell 2009;139:891-906
11. Lammermann T, Sixt M. Mechanical modes of 'amoeboid' cell migration. Curr Opin Cell Biol 2009;21:636-644
12. Danen EH, Sonneveld P, Brakebusch C, Fassler R and Sonnenberg A. The fibronectin-binding integrins alpha5beta1 and alphavbeta3 differentially modulate RhoA-GTP loading, organization of cell matrix adhesions, and fibronectin fibrillogenesis. J Cell Biol 2002;159:1071-1086
13. Rajan N, Habermehl J, Cote M, Doillon CJ, and Mantovani D. Preparation of ready-to-use, storable and reconstituted type I collagen from rat tail tendon for tissue engineering applications. Nat. Protocols 2007;1:2753-2758
14. Haaf F, Sanner A and Straub F. Polymers of N-Vinylpyrrolidone: Synthesis, Characterization and Uses. Polymer J 1985;17:143-152
15. Andrea Ivascu and ManFred Kubbies. Diversity of cell-mediated adhesion in breast cancer spheroids. Int J Oncol. 2007;31:1403-1413
16. Ivascu A, Kubbies M. Rapid generation of single-tumor spheroids for high-throughput cell function and toxicity analysis. J Biomol Screen 2006;11:922-932
17. Bissell, M.J., 1981. The differentiated state of normal and malignant cells or how to define a normal cell in culture (International Review of Cytology 70, 27-100)
18. Alain Beliveau et. 2010 Raf-induced MMP9 disrupts tissue architecture of human breast cells in three-dimensional culture and is necessary for tumor growth in vivo. Genes Dev. 2010 24: 2800-2811
19. Walpita D, Hay E. Studying actin-dependent processes in tissue culture Nat Rev Mol Cell Biol. 2002 Feb;3(2): 137-41.
20. Peppas NA et al. Gel in Biology and Medicine: From Molecular Principles to Bionanotechnology. Adv. Mater. 2006, 18, 1345-1360.
21. Sung KE, Yang N, Pehlke C, Keely PJ, Eliceiri KW, Friedl A, Beebe DJ. Transition to invasion in breast cancer: a microfluidic in vitro model enables examination of spatial and temporal effects. Integr. Bio (camb). 2010 Dec 7.

### Example 2: Integrin control of ZEB/miR-200 balance regulates tumor cell migration strategy and metastatic potential

### Summary

Cellular interactions with the extracellular matrix (ECM) are mediated by transmembrane receptors of the integrin family. These interactions coordinate signal transduction cascades impinging on cell survival, proliferation, and migration. Ablation of the β1 integrin gene attenuates initiation and growth of breast and skin cancers and β1 antibodies sensitize breast tumors in mice to radiotherapy. However, the role of β1 integrins in breast cancer progression from a localized tumor to metastatic disease is unknown. Here, we show that peptide blocking or gene silencing of β1 integrins can cause a shift from cohesive multicellular strand invasion to individual cell migration, which acts pro-metastatic in breast cancer cells. While tumor growth of β1 integrin-depleted breast cancer cells is reduced, intravasation and lung metastasis of cells from these small tumors in an orthotopic mouse model is dramatically enhanced, as is migration in a zebrafish xenograft model. Depletion of β1 integrins alters the balance between miR-200 microRNAs (miRNAs) and ZEB transcriptional repressors leading to a transcriptional downregulation of E-cadherin, which is essential for the induction of individual cell migration and enhanced metastasis. These findings demonstrate that disturbed integrin-mediated interactions with the ECM in cancer cells can alter cell migration strategies and metastatic potential through a miRNA-transcription factor network that controls cell-cell adhesion.

### Results and Discussion

### Silencing β1 integrins induces shift in invasion strategy

We investigated the role of β1 integrins in the invasive/migratory behavior of MCF10a breast epithelial cells and 4T1 breast cancer cells. Cells were microinjected into collagen type I gels where they formed tumor cell spheroids in the first day and cell migration was analyzed over the next 4 days. Wild type MCF10a and 4T1 cells and those expressing control lentiviral shRNAs readily invaded the collagen gel as multicellular strands; a process here referred to as "cohesive invasion" (Fig 13A, 14). Lentiviral shRNAs silencing the β1 integrin gene were transduced followed by two rounds of bulk FACS sorting (Fig 15). In MCF10a, silencing β1 integrins led to a complete block in invasive capacity (Fig 13A). Annexin V labeling was absent from invading strands in MCF10a but occurred to the same extent in the center of wild type and β1 knockdown MCF10a spheroids, suggesting that cell migration rather than cell survival was primarily affected by depletion of β1 integrins in MCF10a (Fig 16). By contrast, cells in 4T1shβ1 spheroids lost the ability to invade as cohesive strands but instead migrated into the collagen as single cells; here referred to as "individual cell migration" (Fig 13A, 14). This switch from cohesive to individual cell migration was accompanied by a loss of E-cadherin-mediated cell-cell junctions (Fig 13B).

We next used a series of snake venom-derived disintegrins and C-lectin type proteins with known integrin specificity to block distinct β1 integrins. Peptides blocking α1β1, α5β1, αvβ3, α4β1 or α9β1 integrins did not alter 4T1 invasive behavior whereas the α2β1-binding peptide, VP12 blocked cohesive invasion and caused cells to adopt an individual cell migration strategy (Fig 13C, 17). Moreover, α2 integrin silencing in 4T1 reduced cohesion and caused a shift to individual cell migration (Fig 13C, 18). The differences in severity of the phenotypes induced by α2 or β1 silencing or α2β1-peptide blocking can probably be explained by variation in the remaining interactions with the ECM.

These findings demonstrate that breast cancer cells can disassemble cell-cell junctions and switch to alternative invasive strategies, when integrin-mediated interactions with the surrounding ECM are disturbed.

### Invasion switch is pro metastatic

Others have described various types of cohesive (sometimes referred to as "collective") or individual cancer cell migration/invasion but it is unknown to what extent these contribute to cancer metastasis. We addressed how the observed switch in invasion strategy induced by silencing β1 integrins would affect metastatic behavior. For this purpose, 4T1 cells were injected in the mammary gland of recipient mice and spontaneous metastasis of the resulting tumors was analyzed. Outgrowth was attenuated for 4T1shβ1 compared to wild type or control shRNA tumors (Fig 19A). Similarly, lung metastases that originated from 4T1shβ1 tumors were much smaller than those from control breast tumors (Fig 19B photographs). These findings are in agreement with earlier studies implicating β1 integrins in breast cancer formation as well as outgrowth of intravenously injected breast cancer cells that have arrested in the lung. However, the small β1 integrin-depleted tumors displayed a dramatic increase in the number of lung metastases, indicating that tumor growth and metastatic potential were inversely affected (Fig 19B). Moreover, the number of circulating tumor cells was strongly increased for 4T1shβ1 tumors as compared to control tumors (Fig 14C). We further analyzed the *in vivo* migratory capacity using an alternative model where tumor cells were injected in zebrafish embryos and spreading throughout the embryo was analyzed. Again, silencing β1 integrins led to an increased ability to migrate away from the primary tumor cell mass and travel to distant sites in the body (Fig 19D-F).

Taken together, these findings demonstrate that the switch from cohesive invasion to individual cell migration that can be induced by interfering with β1 integrin-mediated adhesion, acts pro-metastatic in breast cancer cells.

### E-cadherin expression mediates integrin regulation of invasion

We used affimetrix micro-arrays to compare gene expression profiles in wild type 4T1 cells, the two control short hairpin transduced lines, and the two shβ1 variants. Inginuity Pathway Analysis identified *"cellular movement"* as the process most significantly affected by β1 integrin silencing and also identified *"cell-to-cell signalling"* (Fig 20). Notably, both processes contained the *Cdh1* gene that was significantly downregulated in both shβ1 lines but not in the sh-control line, which was confirmed by qPCR (Fig 21, 20). Indeed, silencing β1 integrins in 4T1 caused a ~75% reduction in E-cadherin surface expression and a similar trend was observed upon α2 silencing (Fig 21 B). β1 silencing also diminished total E-cadherin protein levels, and E-cadherin staining was strongly diminished in primary tumors derived from 4T1shβ1 cells compared to control tumors (Fig 21C,D). Notably, in agreement with their inability to switch to an individual cell mode of migration (Fig 13A), silencing β1 integrins in MCF10a did not affect E-cadherin surface expression (Fig 21 B).

There is evidence that integrin-mediated ECM adhesion can modulate cell-cell adhesion and both positive and negative regulation has been reported but crosstalk at the level of E-cadherin expression has not been demonstrated. We tested if the reduction in E-cadherin expression levels was critically involved in the pro-metastatic switch in cell migration strategy upon β1 integrin silencing. In support of this, restoration of E-cadherin expression to wild type levels in 4T1shβ1 cells reversed the individual cell migration pattern back to cohesive invasion (Fig 21E, 22). Restored expression of E-cadherin also blocked lung metastasis of 4T1shβ1 tumors (Fig 21F) whereas it did not affect tumor growth, which was slow in β1 integrin-depleted tumors irrespective of the absence or presence of E-cadherin, further demonstrating that integrins control tumor growth and metastasis through separate pathways (Fig 21G).

These findings indicate that disturbed β1 integrin-mediated ECM binding attenuates breast tumor growth and may hinder invasion of relatively benign breast cancers whereas in more aggressive tumors that are E-cadherin positive (eg ductal invasive carcinomas) it can promote a switch from cohesive invasion to pro-metastatic individual cell migration that involves a downregulation of E-cadherin expression.

### Integrin regulation of miR-200/2EB balance controls E-cadherin expression

Having established that the ability of β1 integrins to control E-cadherin levels can critically affect metastatic behavior, we next investigated the mechanism by which β1 integrins control E-cadherin levels. Luciferase reporter assays showed that β1 integrin silencing led to a ~80% transcriptional downregulation of the *CdhI* gene (Fig 23A). This prompted us to investigate regulation of a group of E-cadherin transcriptional repressors, including members of the Snail, bHLH, and ZFH families that are implicated in epithelial-mesenchymal transition (EMT). Further analysis of the micro-array data showed that of these repressors, only ZEB2 (also known as Sip1) was significantly (albeit only ~2-fold) and specifically upregulated in both 4T1shβ1 lines and qPCR confirmed the induction of Zeb2, but not Zeb 1, upon β1 integrin silencing (Fig 22B, 23, 20). Both Zeb1 and Zeb2 act as transcriptional repressors of miRNAs of the miR-200 family, which are expressed from two clusters on two distinct chromosomes. Vice versa, miR-200 family members post-transcriptionally repress Zeb1 and Zeb2 by targeting their 3' UTRs. This ZEB/miR-200 feedback loop has been implicated in EMT, and alterations in the balance between ZEB and miR-200 may underlie progression of a number of different types of cancer, including breast carcinomas. miRNA profiling indicated a strong downregulation of all five members of the miR-200 family, in β1 integrin-depleted, but not control shRNA cells (Fig 22C, 23).

We investigated the significance of this regulation in the observed β1 integrin-mediated control of the mode of invasion. To this end, we used synthetic and lentiviral systems for expression of miR-200 family members in 4T1sh1β1 cells as well as synthetic short hairpin inhibitors targeting each of the miR-200 species in 4T1 cells. Synthetic or lentiviral expression of any individual miR-200 family member restored cell-cell adhesion in 2D cultures of 4T1shβ1 cells (Fig 23, 22D, 24). On the other hand, none of the short hairpin inhibitors targeting miR-200 family members was able to interfere with cell-cell adhesion in wild type 4T1 cells (not shown). Together, these data point to overlapping functions of the miR-200 family members in this system and demonstrate that downregulation of all five members is required for the observed inhibition of cohesion upon depletion of β1 integrins. Intriguingly, expression of any of the lentiviral miRNA expression constructs was invariably lost within 5 days after GFP sorting, suggesting that the expression of mature miR-200 species caused a growth disadvantage (not shown). In agreement with a central role for Zeb2 in β1 integrin-mediated control of E-cadherin levels, restored cell-cell adhesion upon expression of miR-200 family members in 4T1shβ1 cells was accompanied by a downregulation of Zeb2, but not Zeb1, concomitant with an upregulation of E-cadherin (Fig 23E). Finally, expression of miR-200 family members in β1 integrin depleted cells also fully restored cohesive invasion in 3D collagen (Fig 23F, 25).

Altogether, our findings establish a novel connection between integrin-mediated cell-ECM interactions and E-cadherin-mediated adherens junctions. Interfering with β1 integrin-mediated ECM adhesion attenuates tumor growth but it can also disturb the ZEB/miR-200 balance leading to E-cadherin downregulation and a pro-metastatic switch in migration strategy. E-cadherin expression is almost invariably lost in invasive lobular breast carcinomas but expression is retained in many other types including the common ductal invasive carcinomas. In those cases, a transient, reversible loss of E-cadherin may occur in a minor population of invading cells that will metastasize but this event may go unnoticed in most carcinomas. In addition to altered integrin expression or activity, cell-ECM interactions can be locally disrupted in tumors due to enhanced proteolytic ECM degradation and our findings suggest that this may well lead to such unnoticed transient and reversible E-cadherin downregulation, allowing tumor cells to escape from the primary tumor mass and metastasize to distant organs.

### Methods

### Cell lines and animals

4T1 mouse breast cancer cells and MCF10a human breast mammary epithelial cells were obtained from ATCC and cultured according to the provided protocol. Rag2-/-;γc-/-mice were housed in individually ventilated cages under sterile conditions. Housing and experiments were performed according to the Dutch guidelines for the care and use of laboratory animals. Sterilized food and water were provided ad libitum. Zebrafish were maintained according to standard protocols (http://ZFIN.org). Embryos were grown at 28.5-30°C in egg water (60 µg/ml Instant Ocean Salts). During injection with tumor cells, embryos were kept under anesthesia in 0.02% buffered 3-aminobenzoic acid ethyl ester (Tricaine, Sigma).

### Antibodies and peptides

For FACS, primary antibodies included HMβ1 anti-mouse β1 (BD Pharmingen), AUUB2 anti-human β1, Ha1/29 anti-mouse α2 (BD Pharmingen), or DECMA anti-mouse/human E-cadherin (Sigma-Aldrich). For Western blot, primary antibodies included HMβ1 anti-mouse β1, 36/E-cadh anti-mouse/human E-cadherin (BD Transduction Laboratories), and B-5-1-2 anti-α-tubulin (Sigma). For immunohistochemistry on frozen tumor sections and in fixed collagen gels, 36/E-cadh anti-mouse/human E-cadherin antibody (BD Transduction Laboratories) was used. For tumor cell migration interference studies, snake venom-derived disintegrins and C-lectin type proteins, including Obustatin (α1β1), Vlo-4 (α5β1; αvβ3), Vlo-5 (β4β1; α9β1) and VP-12 (α2β1), were used at a concentration of 4.6 µM (ref marcinkiewics).

### Stable cDNA and shRNA expression

4T1 and MCF10a cells were transduced using lentiviral shRNA vectors [LentiExpress™; Sigma-Aldrich) according to the manufacturers' procedures and selected in medium containing 2 ∼µg/ml puromycin. Control vectors included shRNA targeting TurboGFP (shc#I) and shRNA targeting eGFP (shc#2). shRNAs silencing mouse β1 integrin included those targeting *gcacgatgtgatgatttagaa* (SEQ ID No: 1) (shp31#1; nucleotides 363-383 in the mouse *Itgb1* coding sequence) and *gccattactatgattatcctt* (SEQ ID No: 2) (shβ1#2; nucleotides 1111-1131 in the mouse *Itgb1* coding sequence). shRNAs silencing mouse α2 integrin included those targeting *gcgttaattcaatatgccaat* (SEQ ID No: 3) (shα2#1; nucleotides 733-753 in the mouse *Itga2* coding sequence) and *gcagaagaatatggtggtaaa* (SEQ ID No: 4) (shα2#2; nucleotides 2274-2294 in the mouse *Itga2* coding sequence). 4T1shβ1 cells were transduced with pCSCG/mECAD lentiviral cDNA expression vector for mouse E-cadherin (provided by Dr. Patrick Derksen, University Medical Center, Utrecht NL). Cells transduced with integrin shRNAs or E-cadherin cDNA were selected for stable knockdown or stable expression phenotypes, respectively by two rounds of bulk FACS sorting (see below for technical details).

### Expression of synthetic siRNA, miRNA Mimics, and miRNA Hairpin inhibitors

Cells were seeded at 5x10⁵ cells per well in 12 wells plates and transfected at a final concentration of 50 nM of siRNA smartpools (Thermo Fisher Scientific; non-targeting control, mouse ZEB1, mouse ZEB2), miRIDIAN miRNA Mimics (Thermo Fisher Scientific; control non-targeting, miR-200a, miR-200b, miR-200c, miR-141, and miR-205), or miRNA Hairpin inhibitors (Thermo Fisher Scientific; control non-targeting, miR-200a, miR-200b, miR-200c, miR-141, and miR-205) using DharmaFECT2 (Thermofisher Scientific). Cells were replated 24 hours post transfection and used for E-cadherin FACS, qPCR analysis, or collagen invasion 48 hours later.

### Luciferase reporter assay

4T1 wild type and 4T1shβ1 cells were transiently transfected with 10 ng of an E-cadherin firefly luciferase reporter plasmid (ref; provided by Dr. Geert Berx, VIB, Gent BE) and 2 ng of a CMV-renilla luciferase reporter using lipofectamine plus (Invitrogen) and analyzed using a dual luciferase kit (Promega) 3 days later, according to the manufacturers' procedure.

### 3D invasion assays

Cell suspensions in culture medium containing 2% polyvinylpyrrolidone (PVP; Sigma-Aldrich) were microinjected using an air driven microinjector (20 psi, PV820 Pneumatic PicoPump; World Precision Inc) into collagen gels prepared from 2.5 mg/ml acid-extracted rat tail collagen type 1. Tumor cell spheroids were monitored for 4 days. For labeling of phosphatidyl serines exposed in the outer leaflet of the membrane of dead cells, gels were incubated overnight with 1:1000 Alexa 488-conjugated Annexin V prepared as described (ref jordi). For immunostaining at 4 days post-injection, gels were incubated for 30 min with 5 µg/ml collagenase (from Clostridium histolyticum, Boehringer Mannheim) at room temperature, fixed with 4% paraformaldehyde, permeabilized in 0.2% Triton X-I 00, and blocked with 1% BSA. Gels were incubated with Rhodamin-conjugated Phalloidin or with E-cadherin antibody followed by Alexa 488-conjugated secondary antibody and Hoechst nuclear staining. Preparations were mounted in Aqua-Poly/Mount solution (Polysciences, Inc) and analyzed using a Nikon TE2000 confocal microscope. Z-stacks (50 x 1 µm) were obtained using a 20x dry objective and converted into a single Z projection using the "extended depth of field" plugin from ImageJ software.

For real time imaging, ∼3 hours time-lapse movies of spheroids were obtained starting at 48 hours post-injection. Image acquisition was performed using a Nikon TE2000 confocal microscope with a temperature and CO₂ controlled incubator. Differential interference contrast (DIC) time-lapse videos were recorded using a charged coupled device (CCD) camera controlled by NIS Element Software. Images were converted into a single avi file in Image-Pro Plus (Version 5.1 ; Media Cybernetics).

### Lentiviral expression of miRNA shMimics

4T1shβ1 cells were transduced using miRIDIAN lentiviral particles expressing mature miRNAs (non-targeting control, miR-200a, miR-200b, miR-200c, miR-141, and miR-205; Thermofisher Scientific) according to the manufacturers' procedures followed by two rounds of bulk sorting for GFP expression. Subsequently, cells were used for E-cadherin FACS, qPCR analysis, or collagen invasion studies.

### Mouse orthotopic transplantation experiments

1 x 10⁵ tumor cells in 0.1 mL PBS were injected into the fat pad of 8-12-week old female Rag2^{-/-};γc^{-l-} mice. Size of the primary tumors was measured using calipers. Horizontal *(h)* and vertical *(v)* diameters were determined and tumor volume *(V)* was calculated: *V*= 4/3π{1/2[√(*h* x *v*)]³}. After 3-4 weeks, animals were anesthetized with pentobarbital and primary tumor and lungs were excised. Primary tumor and left lung were divided into two pieces that were snap frozen in liquid nitrogen for E-cadherin immuno-staining or fixed in 4% paraformaldehyde for H&E staining. For counting of lung metastases, right lungs were injected with ink solution, destained in water, and fixed in Feketes [4.3% (vol/vol) acetic acid, 0.35% (vol/vol) formaldehyde in 70% ethanol]. To analyze circulating tumor cells in some mice blood was drawn from the right atrium via heart puncture after anesthetizing but before excision of primary tumor and lungs. 0.2 ml of blood was plated into 60-mm tissue culture dishes filled with growth medium. After 5 days, tumor cell clones were stained using MTT (Sigma) and counted using Image].

### Zebrafish xenotransplantation experiments

Tumor cells were labeled with CM-Dil (Invitrogen), mixed with 2% PVP, and injected into the yolk sac of enzymatically dechorionated, two-day old Fli-GFP transgenic zebrafish embryos using an air driven microinjector (20 psi, PV820 Pneumatic PicoPump; World Precision Inc). Embryos were maintained in egg water at 34°C for 6 days and subsequently fixed with 4% paraformaldehyde. Imaging was done in 96 well plates containing a single embryo per well using a Nikon Eclipse Ti confocal laser scanning microscope. Z stacks (15 x 5 µm) were obtained using a Plan Apo 4X Nikon dry objective with 0.2 NA and 20 WD. Images were converted into a single Z projection in Image-Pro Plus (Version 6.2; Media Cybernetics). Automated quantification of tumor cell spreading per embryo was carried out using an in-house built Image-Pro Plus plugin.

### mRNA and miRNA analysis

Total RNA for qPCR and miRNA profiling was extracted using Trizol (Invitrogen). cDNA was randomly primed from 50 ng total RNA using iScript cDNA synthesis kit (BioRad) and real-time qPCR was subsequently performed in triplicate using SYBR green PCR (Applied Biosystems) on a 7900HT fast real-time PCR system (Applied Biosystems). The following qPCR primer sets were used: β-action, forward *aacctggaaaagatgacccagat* (SEQ ID No: 5) reverse *cacagcctggatggctacgta* (SEQ ID No: 6); E-cadherin, forward *atcctcgccctgctgatt* (SEQ ID No: 7) reverse *accaccgttctcctccgta* (SEQ ID No: 8); Zeb1, forward *ccttcaagaaccgctttctgtaaa* (SEQ ID No: 9) reverse *cataatccacaggttcagttttgatt* (SEQ ID No: 10); Zeb2, forward *cagcagcaagaaatgtattggtttaa* (SEQ ID No: 11), reverse *tgtttctcattcggccatttact* (SEQ ID No: 12). Data were collected and analysed using SDS2.3 software (Applied Biosystems). Relative mRNA levels after correction for β-actin control mRNA, were expressed using 2^(-ΔΔCt) method.

Detection of mature miRNAs was performed using Taqman microRNA assay kit according to the manufacturer's instructions (Applied Biosystems). The U6 small nuclear RNA was used as internal control.

For micro-arrays, total RNA was extracted using mirVana RNA isolation kit (Ambion Inc). RNA quality and integrity was assessed with Agilent 2100 Bioanalyzer system (Agilent technologies). The Affymetrix 3' IVT-Express Labeling Kit was used to synthesize Biotin-labeled cRNA and this was hybridized to an Affimetrix MG430 PM Array plate. Data quality control was performed with Affymetrix Expression Console v1.I and all raw data passed the affimetrix quality criteria. Median normalization of raw expression data and identification of differentially expressed genes using a random-variance t-test was performed using BRBarray tools (http://linus.nci.nih.gov/BRB-ArrayTools.html). Annotation was done according to the NetAffx annotation date release 2009-11-23. Corrections for multiple testing were made according to the method of Benjamini & Hochberg and false discovery rates (FDRs) are presented.

### Western blot and flow cytometry

For Western blot, cells were lysed with modified RIPA buffer (150 mM NaCl, 1.0% triton-X 100, 0.5% Na deoxycholate, 0.1% 50mM Tris pH 8, and protease cocktail inhibitor (Sigma-Aldrich)). Samples were separated on SDS PAGE gels and transferred to PVDF membranes (Millipore), incubated with primary antibodies followed by horseradish peroxidase-labeled secondary antibodies (Jackson ImmunoResearch Laboratories Inc), and developed with enhanced chemiluminescence substrate mixture (ECL plus, Amersham, GE Healthcare). Blots were scanned on a Typhoon 9400 (GE Healthcare).

For flow cytometry, cells were detached either using trypsin/EDTA (in the case of GFP or integrin surface expression) or by 0.02% EDTA only (in the case of E-cadherin surface expression). Surface expression levels were determined using primary antibodies, followed by fluorescence-conjugated secondary antibodies, and analysis on a FACSCanto or sorting on a FACSCalibur (Becton Dickinson).

### Statistical analysis

The data are presented as mean ± SEM unless otherwise stated. Student's t test (two-tailed) was used to compare groups.

### Example 3: Generation of multicellular spheroids for a range of cell types using microinjection method

Using the same methodology as described in Example 1 (see also Truong et al 2012, Biomaterials 33(1): 181-8), we have generated multicellular spheroids for primary cervical cancer biopsy cells and human breast cell lines including BT20, MDA-MB-435s, HCC1143, HCC1954, SUM149PT, SUM229PE, EVSA-T and SKBR7.

### Example 4: Assessing gene function in multicellular spheroids

As described above, it is appreciated that by exposing multicellular spheroids to one or more chemical inhibitors, one can assess gene function. For example, Example 1 demonstrates that a ROCK inhibitor blocks collective 4T1 cell spheroid outgrowth. Using the same methodology, we have also shown that an inhibitor of Syk blocks PC3 cell spheroid outgrowth. In addition to chemical inhibitors, peptides, synthetic siRNA molecules and shRNA constructs (eg plasmids, adenoviral, retroviral or lentiviral) may be used to assess gene function in similar assays. For example, we have demonstrated that disintegrin peptide blocks collective 4T1 cell spheroid outgrowth, that silencing genes in the TGF-B pathway via siRNA modulates 4T1 cell spheroid outgrowth, and that silencing integrins, Ron or Syk via shRNA constructs can block PC3 cell spheroid outgrowth.

It will be also understood that by scaling up the methodology, the assay may be used to perform whole genome screening.

## Claims

1. A method for producing a multicellular spheroid comprising injecting a cell suspension into a gel wherein the cells are suspended in a carrier material.

2. A method according to Claim 1, wherein the carrier is a bioactive carrier.

3. A method according to any of Claims 1-2, wherein the multicellular spheroid comprises any of stem cells, progenitor cells, somatic cells and bacterial cells.

4. A method according to any of Claims 1-3, wherein the multicellular spheroid comprises cells from a biological sample taken from a subject, such as a biological tissue or a cell-containing fluid.

5. A method according to any of Claims 1-3, wherein the multicellular spheroid comprises immortalised cells.

6. A method according to any of Claims 1-5, wherein the gel is a hydrogel.

7. A method according to any of Claims 1-6, wherein the method comprises producing multiple multicellular spheroids by injecting at different sites in one or more gels.

8. A method according to any of Claims 1-7, wherein the method is automated or semi-automated.

9. A method according to any of Claims 1-8, wherein the gel is in a multi-well plate.

10. A method of producing a gel comprising one or more multicellular spheroids, the method comprising injecting a cell suspension into a gel, wherein the cells are suspended in a carrier material.

11. A gel, comprising two or more multicellular spheroids which are at predefined positions in the gel; optionally wherein the two or more multicellular spheroids have a homogeneous spherical shape with an average diameter between 50 and 2000 µm such as between 200 and 500 µm, optionally wherein at least one of the antigen profile, genetic profile, tumour biology, tumour architecture, cell proliferation rate(s), tumour microenvironment, therapeutic resistance, cell composition, gas concentrations, cytokine expression, growth factor expression and cell adhesion profile of the two or more multicellular spheroids is substantially identical to that of the tissue of origin.

12. A gel comprising at least two multicellular spheroids at predefined positions in the gel wherein at least two multicellular spheroids have different cell compositions.

13. A method of assessing the property of a cell selected from any of survival, growth, proliferation, differentiation, migration, morphology, signalling, metabolic activity, gene expression and cell-cell interaction, the method comprising (i) producing a multicellular spheroid according to any of Claims 1-9, or producing a gel according to Claim 10, or providing a gel according to any of Claims 11 and 12; and (ii) assessing the property of the cell in the multicellular spheroid; optionally wherein the cell is a cancer cell, a stem cell, an endothelial cell, an immune cell or a bacterial cell.

14. A method of assessing the effect of an agent on the property of a cell selected from any of survival, growth, proliferation, differentiation, migration, morphology, signalling, metabolic activity, gene expression and cell-cell interaction, the method comprising (i) producing a multicellular spheroid according to any of Claims 1-9, or producing a gel according to Claim 10, or providing a gel according to any of Claims 11 and 12; and (ii) assessing the effect of the agent on the property of a cell in the multicellular spheroid; optionally wherein the cell is a cancer cell, a stem cell, an endothelial cell, an immune cell or a bacterial cell.

15. Use of a gel according to any of Claims 11-12 in assessing the property of a cell selected from any of survival, growth, proliferation, differentiation, migration, morphology, signalling, metabolic activity, gene expression and cell-cell interaction, or in assessing the effect of an agent on the property of a cell selected from any of survival, growth, proliferation, differentiation, migration, morphology, signalling, metabolic activity, gene expression and cell-cell interaction; or in tissue engineering.

## Patentansprüche

1. Verfahren zur Herstellung eines multizellulären Sphäroids, umfassend Injizieren einer Zellsuspension in ein Gel, wobei die Zellen in einem Trägermaterial suspendiert sind.

2. Verfahren nach Anspruch 1, wobei der Träger ein bioaktiver Träger ist.

3. Verfahren nach einem der Ansprüche 1-2, wobei das multizelluläre Sphäroid bliebige der Folgenden umfasst: Stammzellen, Progenitorzellen, Körperzellen und Bakterienzellen.

4. Verfahren nach einem der Ansprüche 1-3, wobei das multizelluläre Sphäroid Zellen aus einer biologischen Probe umfasst, die aus einem Subjekt entnommen wurde, wie beispielsweise aus einem biologischen Gewebe oder einer zellhaltigen Flüssigkeit.

5. Verfahren nach einem der Ansprüche 1-3, wobei das multizelluläre Sphäroid immortalisierte Zellen umfasst.

6. Verfahren nach einem der Ansprüche 1-5, wobei das Gel ein Hydrogel ist.

7. Verfahren nach einem der Ansprüche 1-6, wobei das Verfahren die Herstellung von mehreren multizellulären Sphäroiden umfasst, indem bei einem oder mehreren Gelen an verschiedenen Stellen eine Injektion durchgeführt wird.

8. Verfahren nach einem der Ansprüche 1-7, wobei das Verfahren automatisiert oder halbautomatisiert ist.

9. Verfahren nach einem der Ansprüche 1-8, wobei sich das Gel in einer Multiwellplatte befindet.

10. Verfahren zur Herstellung eines Gels, umfassend ein multizelluläres Sphäroid oder mehrere multizelluläre Sphäroide, wobei das Verfahren die Injektion einer Zellsuspension in ein Gel umfasst, wobei die Zellen in einem Trägermaterial suspendiert sind.

11. Gel, umfassend zwei oder mehrere multizelluläre Sphäroide, die sich an zuvor festgelegten Positionen in dem Gel befinden; wahlweise wobei die beiden oder mehreren Sphäroide eine einheitliche sphärische Form mit einem mittleren Durchmesser zwischen 50 und 2000 µm, wie beispielsweise zwischen 200 und 500 µm, aufweisen; wahlweise wobei mindestens ein Antigenprofil, ein genetisches Profil, eine Tumorbiologie, eine Tumorarchitektur, (eine) Zellproliferationsrate(n), eine Tumormikroumgebung, eine therapeutische Resistenz, eine Zellzusammensetzung, Gaskonzentrationen, eine Zytokinexpression, eine Expression von Wachstumsfaktor und ein Zelladhäsionsprofil der beiden oder mehrerer multizellulärer Sphäroide im Wesentlichen identisch mit dem Ursprungsgewebe ist.

12. Gel, umfassend mindestens zwei multizelluläre Sphäroide an zuvor festgelegten Positionen in dem Gel, wobei mindestens zwei multizelluläre Sphäroide verschiedene Zellzusammensetzungen haben.

13. Verfahren zur Bewertung der Eigenschaft einer Zelle, die ausgewählt ist aus Überleben, Wachstum, Proliferation, Differenzierung, Migration, Morphologie, Signalweg, Stoffwechselaktivität, Genexpression und Wechselwirkung von Zelle zu Zelle, wobei das Verfahren (i) das Herstellen eines multizellulären Sphäroids nach einem der Ansprüche 1-9 oder das Herstellen eines Gels nach Anspruch 10 oder das Bereitstellen eines Gels nach einem der Ansprüche 11 und 12; sowie (ii) das Bewerten der Eigenschaft der Zelle in dem multizellulären Sphäroid umfasst; wobei die Zelle wahlweise eine Krebszelle, eine Stammzelle, eine Endothelzelle, eine Immunzelle oder eine Bakterienzelle ist.

14. Verfahren zum Bewerten der Wirkung eines Mittels auf die Eigenschaft einer Zelle, die ausgewählt ist aus Überleben, Wachstum, Proliferation, Differenzierung, Migration, Morphologie, Signalweg, Stoffwechselaktivität, Genexpression und Wechselwirkung von Zelle zu Zelle, wobei das Verfahren (i) das Herstellen eines multizellulären Sphäroids nach einem der Ansprüche 1-9 oder das Herstellen eines Gels nach Anspruch 10 oder das Bereitstellen eines Gels nach einem der Ansprüche 11 und 12 umfasst; sowie (ii) das Bewerten der Wirkung eines Mittels auf die Eigenschaft einer Zelle in dem multizellulären Sphäroid, wobei die Zelle wahlweise eine Krebszelle, eine Stammzelle, eine Endothelzelle, eine Immunzelle oder eine Bakterienzelle ist.

15. Verwenden eines Gels nach einem der Ansprüche 11-12 zum Bewerten der Eigenschaft einer Zelle, die ausgewählt ist aus Überleben, Wachstum, Proliferation, Differenzierung, Migration, Morphologie, Signalweg, Stoffwechselaktivität, Genexpression und Wechselwirkung von Zelle zu Zelle, oder die Bewertung der Wirkung eines Mittels auf die Eigenschaft einer Zelle, die ausgewählt ist aus Überleben, Wachstum, Proliferation, Differenzierung, Migration, Morphologie, Signalweg, Stoffwechselaktivität, Genexpression und Wechselwirkung von Zelle zu Zelle; oder zur Gewebekonstruktion.

## Revendications

1. Procédé de production d'un sphéroïde multicellulaire comprenant les étapes qui consistent à injecter une suspension cellulaire dans un gel, dans lequel les cellules sont mises en suspension dans une substance vectrice.

2. Procédé selon la revendication 1, dans lequel la substance vectrice est un vecteur bioactif.

3. Procédé selon l'une quelconque des revendications 1 à 2, dans lequel le sphéroïde multicellulaire comprend des cellules choisies dans le groupe constitué par les cellules souches, les cellules progénitrices, les cellules somatiques et les cellules bactériennes.

4. Procédé selon l'une quelconque des revendications 1 à 3, dans lequel le sphéroïde multicellulaire comprend des cellules provenant d'un échantillon biologique prélevé chez un patient, tel qu'un tissu biologique ou un liquide contenant des cellules.

5. Procédé selon l'une quelconque des revendications 1 à 3, dans lequel le sphéroïde multicellulaire comprend des cellules immortalisées.

6. Procédé selon l'une quelconque des revendications 1 à 5, dans lequel le gel est un hydrogel.

7. Procédé selon l'une quelconque des revendications 1 à 6, lequel procédé comprend l'étape qui consiste à produire plusieurs sphéroïdes multicellulaires en injectant dans différents sites dans un ou plusieurs gels.

8. Procédé selon l'une quelconque des revendications 1 à 7, lequel procédé est automatisé ou semi-automatisé.

9. Procédé selon l'une quelconque des revendications 1 à 8, dans lequel le gel est une plaque à multi-puits.

10. Procédé de production d'un gel comprenant un ou plusieurs sphéroïdes multicellulaires, ledit procédé comprenant l'étape qui consiste à injecter une suspension cellulaire dans un gel, dans lequel les cellules sont mises en suspension dans une substance vectrice.

11. Gel, comprenant deux sphéroïdes multicellulaires ou plus qui sont situés en des positions prédéfinies dans le gel ; facultativement, dans lequel les deux sphéroïdes multicellulaires ou plus ont une forme sphérique homogène d'un diamètre moyen compris entre 50 et 2 000 µm, tel qu'entre 200 et 500 µm, facultativement dans lequel au moins une des caractéristiques choisies dans le groupe constitué par le profil antigénique, le profil génétique, la biologie tumorale, l'architecture tumorale, le(les) taux de prolifération cellulaire, le micro-environnement tumoral, la résistance thérapeutique, la composition cellulaire, les concentrations gazeuses, l'expression des cytokines, l'expression du facteur de croissance et le profil d'adhésion cellulaire des deux sphéroïdes multicellulaires ou plus est substantiellement identique à celle du tissu d'origine.

12. Gel comprenant au moins deux sphéroïdes multicellulaires situés en des positions prédéfinies dans le gel, dans lequel au moins deux sphéroïdes multicellulaires ont des compositions cellulaires différentes.

13. Procédé d'évaluation de la propriété d'une cellule, ladite propriété étant choisie dans le groupe constitué par la survie, la croissance, la prolifération, la différenciation, la migration, la morphologie, la signalisation, l'activité métabolique, l'expression génique et l'interaction cellule-cellule, ledit procédé comprenant les étapes qui consistent à (i) produire un sphéroïde multicellulaire selon l'une quelconque des revendications 1 à 9, ou produire un gel selon la revendication 10, ou fournir un gel selon l'une quelconque des revendications 11 et 12 ; et (ii) évaluer la propriété de la cellule dans le sphéroïde multicellulaire ; facultativement dans lequel la cellule est une cellule cancéreuse, une cellule souche, une cellule endothéliale, une cellule immunitaire ou une cellule bactérienne.

14. Procédé d'évaluation de l'effet d'un agent sur la propriété d'une cellule, ladite propriété étant choisie dans le groupe constitué par la survie, la croissance, la prolifération, la différenciation, la migration, la morphologie, la signalisation, l'activité métabolique, l'expression génique et l'interaction cellule-cellule, ledit procédé comprenant les étapes qui consistent à (i) produire un sphéroïde multicellulaire selon l'une quelconque des revendications 1 à 9, ou produire un gel selon la revendication 10, ou fournir un gel selon l'une quelconque des revendications 11 et 12 ; et (ii) évaluer l'effet de l'agent sur la propriété d'une cellule dans le sphéroïde multicellulaire ; facultativement dans lequel la cellule est une cellule cancéreuse, une cellule souche, une cellule endothéliale, une cellule immunitaire ou une cellule bactérienne.

15. Utilisation d'un gel selon l'une quelconque des revendications 11 et 12 dans l'évaluation de la propriété d'une cellule, ladite propriété étant choisie dans le groupe constitué par la survie, la croissance, la prolifération, la différenciation, la migration, la morphologie, la signalisation, l'activité métabolique, l'expression génique et l'interaction cellule-cellule, ou dans l'évaluation de l'effet d'un agent sur la propriété d'une cellule choisie dans le groupe constitué par la survie, la croissance, la prolifération, la différenciation, la migration, la morphologie, la signalisation, l'activité métabolique, l'expression des gènes et l'interaction cellule-cellule ; ou en ingénierie tissulaire.


### SEQUENCE LISTING

<110> Universiteit Leiden
<120> Methods
<130> LEIBP/P51177PC
<140> PCT/EP2012/055726
   <141> 2012-03-29
<150> GB - 1105226.3
   <151> 2011-03-29
<160> 13
<170> PatentIn version 3.5
<210> 1
   <211> 21
   <212> RNA
   <213> Artificial
<220>
   <223> shRNA
<400> 1
   gcacgatgtg atgatttaga a 21
<210> 2
   <211> 21
   <212> RNA
   <213> Artificial
<220>
   <223> shRNA
<400> 2
   gccattacta tgattatcct t 21
<210> 3
   <211> 21
   <212> RNA
   <213> Artificial
<220>
   <223> shRNA
<400> 3
   gcgttaattc aatatgccaa t 21
<210> 4
   <211> 21
   <212> RNA
   <213> Artificial
<220>
   <223> shRNA
<400> 4
   gcagaagaat atggtggtaa a 21
<210> 5
   <211> 23
   <212> DNA
   <213> Artificial
<220>
   <223> PCR Primer
<400> 5
   aacctggaaa agatgaccca gat 23
<210> 6
   <211> 21
   <212> DNA
   <213> Artificial
<220>
   <223> PCR Primer
<400> 6
   cacagcctgg atggctacgt a 21
<210> 7
   <211> 18
   <212> DNA
   <213> Artificial
<220>
   <223> PCR Primer
<400> 7
   atcctcgccc tgctgatt 18
<210> 8
   <211> 19
   <212> DNA
   <213> Artificial
<220>
   <223> PCR Primer
<400> 8
   accaccgttc tcctccgta 19
<210> 9
   <211> 24
   <212> DNA
   <213> Artificial
<220>
   <223> PCR primer
<400> 9
   ccttcaagaa ccgctttctg taaa 24
<210> 10
   <211> 26
   <212> DNA
   <213> Artificial
<220>
   <223> PCR Primer
<400> 10
   cataatccac aggttcagtt ttgatt 26
<210> 11
   <211> 26
   <212> DNA
   <213> Artificial
<220>
   <223> PCR Primer
<400> 11
   cagcagcaag aaatgtattg gtttaa 26
<210> 12
   <211> 23
   <212> DNA
   <213> Artificial
<220>
   <223> PCR Primer
<400> 12
   tgtttctcat tcggccattt act 23
<210> 13
   <211> 5
   <212> PRT
   <213> Artificial
<220>
   <223> Cell adhesion molecule
<400> 13
   Tyr Ile Gly Ser Arg
   1               5